(19) Europäisches Patentamt
European Patent Office
Office européen des brevets

(11) EP 1 842 541 A1

(12) EUROPEAN PATENT APPLICATION

(43) Date of publication:
**10.10.2007 Bulletin 2007/41**

(21) Application number: **06450045.7**

(22) Date of filing: **29.03.2006**

(51) Int Cl.:
***A61K 31/404*** *(2006.01)* ***A61K 31/352*** *(2006.01)*
***A61K 31/366*** *(2006.01)* ***A61K 31/353*** *(2006.01)*
***A61K 36/31*** *(2006.01)* ***A23L 1/30*** *(2006.01)*
***A61P 15/12*** *(2006.01)* ***A61P 25/34*** *(2006.01)*
***A61P 35/00*** *(2006.01)* ***A61P 43/00*** *(2006.01)*

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK YU**

(71) Applicant: **G.I.M.-GESELLSCHAFT FÜR INNOVATIVE MEDIZIN GMBH NFG OHG**
**1090 Vienna (AT)**

(72) Inventors:
- **Jungbauer,Alois**
**1210 Wien (AT)**
- **Medjakovic, Svjetlana**
**1030 Wien (AT)**
- **Zöchling, Alfred**
**2523 Tattendorf (AT)**

(74) Representative: **Sonn & Partner Patentanwälte**
**Riemergasse 14**
**1010 Wien (AT)**

# (54) Plant components and extracts and uses thereof

(57) The present invention provides a medicament comprising a chemical compound according to formula 1:

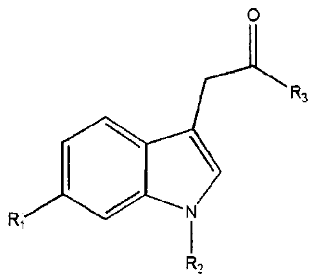

(formula 1)

wherein R1 is hydrogen, fluorine or methoxy, R2 is hydrogen, methyl, ethyl, propyl or fluorine and R3 is furanyl or pyranyl, and its use for the activation of the aryl hydrocarbon receptor, as well as the use of a plant extract or plant juice for the production of a preparation for the activation of the aryl hydrocarbon receptor.

EP 1 842 541 A1

## Description

The present invention relates to plant components and extracts and uses therefor.

The aryl hydrocarbon receptor (AhR), also known as dioxin receptor, is a ligand-activated transcription factor and a member of the basic helix-loop-helix (bHLH) PER-ARNT-SIM (PAS) protein family. This transcription factor has been found in nearly all vertebrates. The molecular mass of the AhR varies from 95 to 105 kDa by species and by strain. The AhR is an orphan receptor; although numerous xenobiotic and natural compounds are known to interact with AhR, a physiological ligand is unknown. Even though evidence for a certain endogenous physiological ligand could not be found so far. In various studies an AhR signalling pathway in absence of exogenous ligands has been demonstrated and a disruption of AhR expression pattern also resulted in decreased development of mouse blastocysts and alternation in cell cycle progression. Examples of diverse ligands include dietary compounds [2-4], natural [5, 6] and synthetic flavonoids [7], as well as anthropogenic chemicals such as dioxins (e.g., 2,3,7,8-tetrachlorodibenzo-p-dioxin) [8, 9], furans and halogenated aromatic hydrocarbons (HAHs) [10]. Among the bHLH/PAS family, AhR is the only member that requires ligand binding for activation.

Activation of AhR leads to an upregulation of the cytochromes p450 CYP1A1, CYP1A2 and CYP1B1 and phase II enzymes thus leading to the metabolism and degradation of foreign substances. The ligand binding domain is circumjacent PAS B. The hsp90 binding domain is an intrinsic component of the ligand binding domain.

A wide range of substances seems to be able to bind to the aryl hydrocarbon receptor (AhR) and thereby excite the expression of particular genes. Common denominator of these compounds is their chemical structure: most of them are planar, aromatic and hydrophobic molecules. But the structural diversity within the AhR-ligands is great [12]. The best known agonist in the literature is certainly dioxin (2,3,7,8-tetrachlorodibenzo-p-dioxin), which is a very potent toxin. A lot of other environmental toxins and anthropogenic substances, termed polycyclic aromatic hydrocarbon (PAHs), have a similar effect. Natural ligands of the AhR belong to the group of flavonoids and isoflavonoids. Flavonoids are secondary plant compounds, which are known to have various bioactivities such as antioxidative and anticarcinogenic properties, and some act also as phytoestrogens. Some flavonoids act as agonist of the AhR while others function as antagonists. A beneficial effect of antagonists is the suppression of the toxicity of dioxin, e.g. This could be proved for chrysin, baicalein, apigenin, kaempferol and naringenin [5, 6].

Other features of the AhR beside its role in xenobiotic metabolism via cytochrome p450 regulation are a result of its dimerisation with HIF 1$\alpha$, and HIF 1$\beta$. The aryl hydrocarbon receptor nuclear translocator (ARNT) is like its dimerisation partner AhR a bHLH-protein [1] and also known as hypoxia-inducing factor 1$\beta$ (HIF 1$\beta$). But in contrast to the AhR, this protein is constitutively localised in the nucleus. ARNT is necessary for the ligand-mediated activation of the AhR within the nucleus, but does not independently bind ligands. In addition, ARNT is not required for AhR translocation into the nucleus in spite of the term "nuclear translocator". This is a relict from the time ARNT was cloned for the first time and misattributed to a cytoplasmatic protein. ARNT acts not only as binding partner for the AhR but also as general partner factor for members of the bHLHPAS family, such as AhR, HIF 1$\alpha$ and SIM (single-minded) proteins. Moreover, ARNT forms homodimers as well as heterodimers with the mentioned bHLH-PAS proteins [11]. In response to low $O_2$ concentration ARNT and HIF 1$\alpha$ form a heterodimer. This hypoxia inducible transcription factor (HIF 1$\alpha$) mediates the regulation of vascular endothelial growth factor [26] and erythropoietin [27] among other things. The last-mentioned is a glycoprotein hormone that regulates proliferation, differentiation and maturation of erythroid cells. HIF 1$\alpha$ is dependent on hypoxia-induced stabilisation. Under normoxic conditions a rapid degradation, which is mediated by the ubiquitin-proteasome system, is noticed. ARNT is not affected by the oxygen partial pressure. According to new data it seems that the observed hypoxia-induced reduction in AhR-mediated gene regulation is not triggered through ARNT occupation [28]. The expression of ARNT seems to be essential for normal development: ARNT knock-out mice die in utero between 9.5 and 10.5 days of gestation [29]. Comprising ARNT appears to be very important for a couple of distinct signalling pathways that are activated by different stimuli.

Furthermore, AhR competes with the oestrogen receptor for the hormone responsive element. Therefore, several components which potentially bind AhR are antioestrogenic, and have primarily been characterised for an antioestrogenic effect, although most antioestrogenic compounds have their effects because of direct interactions with an oestrogen receptor.

The US 2002/0147155 A1 mentions the use of the aryl hydrocarbon receptor binding ligands indole-3-carbinol, indole-4-carbinol, indole-5-carbinol, diindolylmethane, tryptophan, tryptamine, indole acetic acid for treating endometriosis in a woman. The antioestrogenic effect of AhR ligands is used to this end since AhR ligands have been shown to reduce the levels of oestrogen in women.

The US 5,948,808 provides indole-3-carbinols and diindolylmethane for the treatment of oestrogen-dependent tumors, such as breast cancer and mammary cancer.

According to the US 2003/0096855 indole-3-carbinol is an unstable precursor of diindolylmethane which can be isolated from cruciferous vegetables. This publication proposes the use of diindolylmethane for the treatment of common warts and related oral-genital HPV infections.

The EP 1 418 164 A1 describes aryl hydrocarbon receptor ligand antagonists and their use to reduce the harmful effects of halogenated (HAH) and polycyclic (PAH) aryl hydrocarbons which hyperactivate the AhR without inhibition.

In the EP 1 477 178 A2 compositions comprising natural phyto-oestrogens selected from genistein, daidzein, formononetin and biochanin A are provided for cancer treatment, treatment of pre-menstrual syndrome, menopause and hypercholesterolaemia. Such phyto-oestrogens are preferably isoflavones obtained from red clover or soy.

The US 2003/0219499 A1 describes an isoflavone-containing extract of clover or chick peas comprising oestrogenic compounds for a use in oestrogen therapy. Formononetin, biochanin, daidzein and genistein are mentioned as agonists for the human oestrogen receptor therein.

The EP 1 174 144 A1 discloses a plant extract, obtainable by ethanolic extraction, comprising the isoflavonoids genistein, daidzein, formononetin and biochanin A, as having anti-cancer and oestrogen activity.

The WO 99/43335 notes that the anti-cancer isoflavones genistein, daidzein, formononetin and biochanin occur in plants principally in the glycoside form bound to sugars such as glucose. Suitable plants are legumes, including soy, chick peas, lentils, beans, grams and clovers. Soy and clover contain the highest levels of isoflavones. Other polyphenols found in these plants include pratensin, quercetin, coumesterol, resveratrol, and phytic acid. According to this publication the isoflavones are extracted from said plants, the glycosides are removed and the isoflavone product can be used as anti-cancer compounds. The glycosides are removed by hot extraction with a water-organic solvent mixture, preferably at 90°C, with 0.5% to 70% organic solvent, e.g., $C_{1-4}$ alcohols.

Activation of the aryl hydrocarbon receptor has several beneficial effects. Therefore, it is the aim of the present invention to provide easily accessible products for the specific activation of the aryl hydrocarbon receptor.

The present invention provides a medicament comprising a chemical compound according to formula 1:

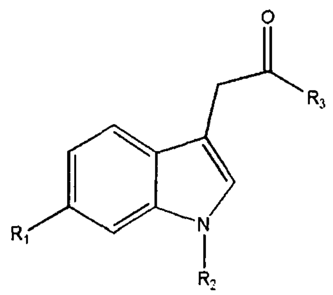

(formula 1)

wherein R1 is hydrogen, fluorine or methoxy, R2 is hydrogen, methyl, ethyl, propyl or fluorine and R3 is furanyl or pyranyl.

Preferably the chemical compound is 1-(2-furanyl)2-(3-indolyl)ethanone (formula 2):

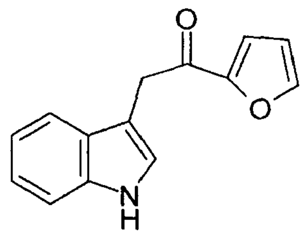

(formula 2).

1-(2-furanyl)2-(3-indolyl)ethanone, synonymous with 2-(Indol-3-yl)acetylfuran ($C_{14}H_{11}NO_2$, molecular weight 225.28 g/mol), is the single most potent AhR activator in sauerkraut and can, for example, be isolated from sauerkraut or sauerkraut juice through chromatographic purification. Prior chemical synthesis methods of this compound are known from [34] and [35]. Chemical synthesis procedures for compounds of formula 1 are also available through the disclosed route via variation of reagents.

The inventors have discovered the presence of 1-(2-furanyl)2-(3-indolyl)ethanone in sauerkraut and its isolation. Therefore, the present invention provides in another aspect a method for the isolation of a compound of formula 2, comprising a chromatographic purification of sauerkraut or sauerkraut juice. Chromatographic purifications are well known in the field of the art, including gradient eluations with various eluens.

The present invention provides a use of biochanin A, formononetin (both form, e.g., red clover, soy, alphalpha), a compound of formula 1 as defined above, preferably 1-(2-furanyl)2-(3-indolyl)ethanone, or mixtures thereof for the production of a pharmaceutical or nutritional preparation for the activation of the aryl hydrocarbon receptor. These compounds can be used as single isolated compounds or can be coextracted (or coisolated) from a plant. Biochanin A and formononetin are known compounds which are also oestrogen receptor agonists according to the US 2003/0219499 A1. This feature has often been used for oestrogenic effects, in cancer treatment and oestrogen regulation, e.g. The present inventors have surprisingly found that biochanin A and formononetin are also ligands of the aryl hydrocarbon receptor (AhR). The use thereof as aryl hydrocarbon receptor activators has certain benefits not considered in the prior art, such as the activation of genes, e.g., cytochrome P450, which leads to a detoxification. Although AhR activators are presumed to function anti-oestrogenic because of the competition of the AhR and the oestrogen receptor for the hormone responsive element, the function of biochanin A and formononetin as oestrogen receptor agonists compensates this effect.

The use of biochanin A, formononetin, a compound of formula 1 as defined above, preferably 1-(2-furanyl)2-(3-indolyl)ethanone, or mixtures thereof, as well as a plant extract, as aryl hydrocarbon receptor activator ex vivo is also provided.

Another preferred use of biochanin A, formononetin, 1-(2-furanyl)2-(3-indolyl)ethanone, or mixtures thereof, is as aryl hydrocarbon receptor activator ex vivo. These compounds can be used to stimulate the AhR in cell cultures for experimental reasons but also to produce products of the AhR transcriptional complexes, such as cytochrome P450. Such products can then be harvested and isolated.

A further preferred use according to the invention is the use of biochanin A, formononetin, a compound of formula 1 as defined above, preferably 1-(2-furanyl)2-(3-indolyl)ethanone, for the production of a preparation for treatment of menopausal disorders, detoxification in smokers, abatement of hypoxia, anti-ageing, cancer treatment or cancer prevention, anti-inflammation and increased wound-healing, especially for selective arylhydrocarbon receptor modulators (SAhRM). Many beneficial effects are associated with activation of the AhR. The detoxification, for example, can lead to a reduction in harmful effects in smokers, e.g., hypoxia due to AhR association with HIF 1$\alpha$. The reduction of harmful compounds further lessens the risk of cancer, the reduction of antigenic compounds by mutated cancerous cells and the reduction of the effects of ageing.

In particular, the preparation is for the treatment of cancer and the cancer is selected from liver cancer, lung cancer, colon cancer, colorectal cancer, breast cancer, prostate cancer, skin cancer, papilloma virus induced cancer, in particular cervical-vaginal cancer.

In another aspect of the present invention a plant extract or plant juice is used for the production of a pharmaceutical or nutritional preparation for the activation of the aryl hydrocarbon receptor, wherein the plant extract or juice comprises aryl hydrocarbon receptor activity. This activity is related to the presence of at least one aryl hydrocarbon receptor ligand or a metabolic precursor of an aryl hydrocarbon receptor ligand. The metabolic precursor can be transformed into an aryl hydrocarbon receptor ligand via metabolism in the digestive system or through cellular metabolism. Aryl hydrocarbon receptor ligands are not selected for their oestrogenic or anti-oestrogenic activity but for their ability to activate the AhR. AhR activation results in the upregulation of associated genes, such as members of the cytochrome P450 family, which leads to the metabolism of foreign compounds and to the detoxification in a patient. Although some isoflavonoids are activators of the AhR, several isoflavonoids, previously selected for their oestrogenic activity, they do not activate the AhR such as daidzein, genistein or quercetin (see table 1 below). Furthermore, the suitability of plant extracts or plant juices used according to the invention is not restricted to plants with constituents, known for their (anti-) oestrogenic activity.

Preferably, the plants are cruciferous vegetables. An especially high AhR stimulative activity has surprisingly been found in this class of plants.

Preferably the plant is a fermented plant, preferably fermented by a lactobacillus. Through fermentation the plant's shelf-life is highly increased in a natural way without chemical preservatives. Although the fermentation and acidious pH degrades known weaken AhR ligands like indole-3-carbinol, the AhR activating activity was still surprisingly high in fermented plants like sauerkraut. Microorganisms for a fermentation process include Lactobacillus lactis, L. plantarum, L. sakei, Leukonostoc mesenteroides, Pediococcus pentosaceus and P. Dextrinicius. Furthermore, it is suspected that the furan ring in the compound of formula 2 is added through fermentation and further processing such as pasteuerisation.

Preferably, the plant is selected from sauerkraut, pepper, black pepper, cress, clover, red clover, pueraria, or mixtures thereof, preferably from sauerkraut or cress. Especially sauerkraut, cress and pepper extracts or juices have a surprisingly high AhR activation potential (cf. example 9). These high activities are not only the result of known constituents which activate the AhR but also include some yet unknown compounds. One of these compounds is the newly found chemical compound 1-(2-furanyl)2-(3-indolyl)ethanone of formula 2. Sauerkraut (fermented cabbage) comprises glucobrassicin, which readily degrades to indole-3-carbinol and 3,3'-diindolylmethane, indolo[3,2-b]carbazole and ascorbigens [21]. Although indole-3-carbinol, indolo[3,2-b]carbazole and diindolylmethane are known as AhR ligands, they

have a low stability and further degrade to nonreactive products. Especially fermenting of cabbage, as, for instance, carried out in sauerkraut, reduces the levels of these glucosinolates to zero [22]. Therefore, sauerkraut has never been considered as a source of AhR ligands before. Surprisingly, it was found that sauerkraut extracts and juices show an extremely high AhR stimulating activity, which cannot be related to these known compounds as was shown by HPLC analysis (figures 4-7, especially figure 11, examples 10-12). The group of flavonoids has also members with strong AhR activity. However, sauerkraut does not contain flavonoids [22]. Therefore, sauerkraut comprises new AhR ligands not known in the prior art. Other plants with a high isoflavone content, such as soy, exhibit only a negligible AhR activation since these plants comprise only flavoinoids with negligible AhR activating potential. Another preferred plant is cress. Cress comprises active compounds of the group of isothiocyanates, such as benzylisothiocyanate (a breakdown product of glucotropaeolin) or phenylisocyanate (a breakdown product of gluconasturtin). However, these compounds are known to have DNA damaging effects in high concentrations (>2.5 μM) [30]. In lower concentrations these compounds stimulate cell chemoprotection via up-regulation of cytochrome P4501A2, glutathione-S-transferase and UDP glucuronosyltransferase (e.g., in garden cress juice [31]). The mechanism for this up-regulation results from binding of isothiocyanates to the antioxidant responsive element or to the xenobiotic responsive element region of the DNA [30]. No effect of cress on the AhR is known.

Preferred plants used according to the invention comprise the compounds apigenin, biochanin A, coumestrol, diindolylmethane, equol, formononetin, indole-3-carbinol, 2-(1'H-indole-3'-carbonyl)-thiazole-4-carboxylic acid (ITE), naringenin, 1-(2-furanyl)2-(3-indolyl)ethanone, or a metabolic precursor thereof. The plant extracts or juices comprise a multitude of several compounds with synergistic effects compared to the use of single compounds. Because the individual concentration of individual components is lower in a mixture with equal activity compared to isolated compounds, side-effects, e.g. oestrogenic effects, are minimized.

Preferably, the preparation as mentioned above is a dry preparation. This allows easy handling, transportation and application of the preparation.

In another preferred embodiment of the present invention the preparation is a concentrated extract or juice, preferably concentrated by a factor 2 to 500, preferably 5 to 400, more preferred 10 to 300, most preferred 80 to 150, e.g. 100, relative to the original plant mass. Such a concentrate can be produced by common techniques, e.g. further extraction (e.g. solid phase extraction (SPE)) and evaporation of the solvent, and results in a preparation with lower mass and higher AhR activating potential.

Even more preferred, the preparation is prepared by the steps of

- obtaining an extraction or juice of the plant comprising an aryl hydrocarbon receptor ligand,
- optionally thickening of the extract or juice, preferably by vacuum evaporation, and/or solid phase extraction, preferably with ethanol,
- drying the extract or juice,
- packaging of the dry product.

The extraction mentioned above can be performed by any method known in the art, e.g. hot alcoholic extraction, but can also be performed using solid phase extraction (cf. examples 6-7).

Preferably, especially if 1-(2-furanyl)2-(3-indolyl)ethanone is present in the plant, the extract is concentrated using solid phase extraction with ethanol. By this method a concentration of the active compound by 500 times is possible. The drying step is then preferably vacuum evaporation at 30-60°C, more preferred 4-55°C, a range in which the active compound is stable.

Preferably, the dry product is packaged into capsules or blister packs.

Preferably, the plant extract or juice has an aryl hydrocarbon receptor activating potential of at least 2000, preferably at least 3000, even more preferred at least 5000, equivalent nmol β-naphtoflavone/g sample. β-naphtoflavone is a very potent agonist of the AhR and qualifies as a good standard or reference for the measurement of AhR activating potential (also referred to as AhR activity herein). Its EC50 value was $2.8x10^{-8}$ mol/l. A "AhR unit" thus equals to an activity of an equivalent amount of β-naphtoflavone in nmol per g sample.

This use of plant extracts or plant juices also includes the production of a preparation for treatment of menopausal disorders, detoxification in smokers, abatement of hypoxia, anti-ageing, cancer treatment or cancer prevention, anti-inflammation and increased wound-healing. Preferably, the treated or prevented (wherein the use is preventive, and an absolute success cannot be guaranteed but the risk of getting cancer is lowered) cancer is breast cancer, colon cancer, lung cancer or prostate cancer. The cancer chemoprevention can be recommended since through the activation of the AhR during strong weight loss apolar compounds are released. These beneficial effects are based on transcriptional activation of the AhR. The anti-inflammatory effects and increased wound-healing are based on downregulation of COX-2 by NFκB mediated by the AhR.

In particular, the plant preparation or the compound of formula 1 is used for the treatment of cancer, especially the cancer is selected from liver cancer, lung cancer, colon cancer, colorectal cancer, breast cancer, prostate cancer,

skin cancer, papilloma virus induced cancer, in particular cervical-vaginal cancer as an example of a papilloma virus induced cancer. The mode of action of the compound of formula 2 and its derivates according to formula 1, or the plant extract as a selective aryl hydrocarbon receptor modulator ultimately inhibits the cell growth of cancer cells. They modulate the cell cycle through cyclin dependent kinases from CDK-family and lead the cell to apoptosis, they downregulate the oncogenes through AhR mediated control of NFκB, immunomodulate and hereby the action of papilloma virus induced cancers, they prevent angiogenesis, the formation of DNA adducts or the prevention of phase I enzyme conversion of compounds into cancerogenic compounds.

As mentioned above, the compounds biochanin A, formononetin or a compound of formula 1, especially 1-(2-furanyl)2-(3-indolyl)ethanone, or the specific plant extracts or juices can be used for the production of cytochrome P450 or glutathione-S-transferase Ya.

A further aspect of the present invention is a method for the activation of the aryl hydrocarbon receptor using a plant extract or plant juice as mentioned above or using biochanin A, or formononetin or a compound of formula 1 as defined above, preferably 1-(2-furanyl)2-(3-indolyl)ethanone as aryl hydrocarbon receptor activator as mentioned above.

The present invention is further illustrated by the following figures and examples without being limited thereto.

Figures:

Figure 1: Schematic illustration of the yeast strain YCM3 used for AhR activation assays.

Figure 2: Logistic dose response curves of some polyphenols, in comparison to β-naphtoflavone (A). Logistic dose response curves of indole-3-carbinol and diindolylmethan in comparison to β-naphtoflavone (B).

Figure 3: A: Activation of the AhR by juices (concentration 100fold enriched with solid phase extraction). B: Activation of the AhR by vegetable juices (undiluted). Sauerkraut juices are from different producers: (1) Alnatura, (2) NaturAktiv, (3) Biotta. Assayed in microtiterplates.

Figure 4: Reversed phase HPLC $C_{18}$ of sauerkraut juice (100-fold enriched with SPE).

Figure 5: Transactivating potential of the HPLC fractions of sauerkraut juice on the AhR. Fractions collected every 2 minutes (fraction number multiplicated with 2 = retention time).

Figure 6: AhR activity of fractions after minute 50 of the sauerkraut HPLC collected in 0.5 min time intervals measured by the yeast assay. Peak activity was measured between minutes 52-53,5 (fractions 5-7).

Figure 7: Reversed phase HPLC $C_{18}$ of indole-3-carbinol (eluates at minute 70) and diinolylmethane (eluates at minute 67). The grey arrow indicates the time when the highest AhR activity was measured in the sauerkraut HPLC (minute 52-53).

Figure 8: Mean LDR of beta-Naphtoflavon with standard deviation.

Figure 9: Change of activity of the sauerkraut juice during SPE-purification. The AhR yeast assay was performed in 3 dilution steps (pure, 6:10, 2:10).

Figure 10: Activity of the re-diluted 100fold concentrated sauerkraut juice. (Concentration 1 is the undiluted 100fold concentrated juice).

Figure 11: AhR-activities of the sauerkraut juice fractions (100fold enrichment with SPE). Column 1 (C1, fractions 1 to 6 F1-F6): conditioning with acetone/hexane, elution with acetone and dissolving of the dried remainder in DMSO. Column 2 (C2, fractions 1 to 10 F1-F10): all steps with ethanol. Column 3 (C3, fractions 1 to 10 F1-F10): like column 2, except for the dissolving step, that was done with DMSO.

Figure 12: A: Comparison of the retention times of the single compounds indole-3-carbinol, 3,3'-dindolylmethane, indolo-[3,2-b]carbazole, tryptanthrin and compound "X" (1-(2-furanyl)2-(3-indolyl)ethanone), measured in 5 HPLC-runs. B: RP-HPLC of the mixture of indole-3-carbinol, 3,3'-dindolylmethane, indolo-[3,2-b]carbazole, tryptanthrin and compound "X" within one run.

Figure 13: Impact of ph on the transactivating potential of sauerkraut juice.

Figure 14: Impact of the temperature on the transactivating potential of sauerkraut juice.

Figure 15: AhR-activity of compound "X" compared to β-naphtoflavone.

Figure 16: Analytical RP-HPLC-analysis of the sauerkraut juice (100fold enriched with SPE), which was used for the preparative RP-HPLC.

Figure 17: Quality control of purification of compound "X" (fraction 52).

Figure 18: ER-LBA of 100fold concentrated sauerkraut juice in comparison to E2 on ERα and ERβ.

Figure 19: Schema of the identification of the most active fraction of sauerkraut juice.

**Examples:**

**Example 1: Yeast AhR screen**

The yeast AhR screen is an in-vitro test to detect transactivating potential of substances on the arylhydrocarbon receptor. The recombinant yeast strain YCM3 of *Saccharomyces cerevisiae,* which was a present of Charles Miller, was used. The assay procedure was essentially as described by Miller [13]. The human AhR and ARNT genes are integrated into chromosome III of the yeast. The galactose-regulated GAL 1,10 promotor is accountable for the expression of AhR and ARNT in equal shares by induction with galactose, the carbon source of the medium (GOLD medium -trp). If a ligand of the AhR is present, an AhR/ARNT heterodimer is built and can bind to the five xenobiotic response elements of the lacZ-reporter plasmid and induce the expression of the lacZ-gene. Therefore the transcriptional activation of a ligand is quantified via β-galactosidase activity. A schematic diagram of the genetic properties of YCM3 is displayed in 2. The yeast AhR assay was performed in 50 ml plastic tubes with conical bottom and in microtiterplates.

**Example 2: Assay in plastic tubes**

A colony of YCM3 was transferred in SCM -trp medium and grown to OD600 of about 2.0. For each experiment a new overnight culture was prepared in SCM -trp medium. For this purpose a 1:10 dilution of the YCM3 yeast stock in SCM -trp medium was prepared. After incubation overnight the culture was diluted to an OD600 of 0.4 and grown again to an OD600 of 1.0 to 1.5. For the assay the culture was 1:50 diluted in Gold galactose -trp media, which contains 1.2% galactose as the inductor. 50 μl of test substance (dissolved in DMSO) and 5 ml of this yeast culture were incubated for 17 h at 30°C while shaking. The tubes have to be in an inclined position for optimal yeast growth. For the blank only 50 μl of DMSO were added to the yeast culture. A calibration curve with β-naphtoflavone was performed within each test run. After incubation cells were collected by centrifugation at 2500 rpm for 5 min. The supernatant was discarded, the pellets were resuspended in 1 ml lacZ-buffer, transferred into 1.5 ml test tubes and centrifuged at 10 000 rpm and 4°C for 5 min. The supernatant was removed again and the pellets were resuspended in 100 μl lacZ buffer. Disintegration of the yeast cells was performed by vortexing with glass beads (3 times for 30 s with a rest on ice of 15 s between the vortexes). After centrifugation at 10 000 rpm and 4°C for 10 min, the clear supernatant was used to perform the β-galactosidase and the protein-assay.

For the β-galactosidase-assay 5 μl from each test tube were transferred to a 96-well microtiterplate. 250 μl of a 1:6 diluted o-nitrophenyl-b-galactopyranoside (ONPG)-solution (dilution with lacZ-buffer) were added to each well. The microtiterplate was incubated at 37°C until a yellow colour had developed. The reaction was stopped by adding 100 μl of 1 M $Na_2CO_3$ and the reaction time was noticed. The absorption was measured at 405 nm (reference wavelength 620 nm). Each determination was carried out in duplicates.

For the protein assay a modified method according to Bradford was performed. 5 μl of the supernatant of each test tube were transferred to the microtiterplate and 250 μl of Bio-Rad protein assay reagent (diluted with water 1:10) were added. The absorption was measured at a wavelength of 570 nm and a reference wavelength of 690 nm. As reference a standard curve with BSA was employed within each test. Each determination was carried out in duplicates.

**Example 3: Assay in microtiterplate**

A colony of YCM3 was transferred in SCM -trp medium and grown to $OD_{600}$ of about 2.0. For each experiment a new overnight culture was prepared in SCM -trp. For this purpose a 1:10 dilution of the YCM3 yeast stock in SCM -trp medium was prepared. After incubation overnight the culture was diluted to an $OD_{600}$ of 0.4 and grown again to an $OD_{600}$ of 1.0 to 1.5. For the assay the culture was 1:50 diluted in Gold galactose -trp media, which contains 1.2% galactose as the inductor.

1 μl of test substance (dissolved in DMSO) and 100 μl of the yeast culture in galactose media were transferred in the wells (flat bottom) of a 96-well sterile microtiterplate. Each determination was carried out in duplicates. A calibration curve with β-naphtoflavone was performed within each test run. For better results only the inner part of the plate was used for the assay, because of a greater evaporation in the outer wells (row A and H, column 1 and 12). 150 μl of the diluted yeast culture were transferred into the outer wells. The microtiterplate was incubated for 17 h at 30°C and while shaking.

The disintegration of the assay was performed with N-lauroylsarcosine, a strong detergent. A related method was described by Kippert [14], who used 1.5 ml centrifuge tubes and a disintegration with a 0.2% sarcosyl-solution. For the microtiterplate assay a modified version was used and the disintegration was performed via a 0.5% sarcosyl-solution. To circumvent a centrifugation of the plate and a transfer of the supernatant in another plate, what would complicate the assay unnecessarily, the calculation of the AhR-units was related to the optical density of 600 nm, instead of the protein level. A similar proceeding was applied by Adachi et al. [15] and Miller [13].

After incubation 150 µl Z-Sarcosyl-buffer to each well were added. Then the OD was measured at 600 nm. For complete disintegration of the cells the plate was incubated for 20 min at 30°C. Afterwards 50 µl of ONPG-solution (a 1:6 diluted) were added to each well and incubated at 37°C until a yellow colour had developed. The reaction was stopped by adding of 50 µl 1 M $Na_2CO_3$ and the reaction time was noticed. The absorption was measured at 405 nm (reference wavelength 620 nm).

**Example 4: Evaluation**

The expression of β-galactosidase is equivalent to the quantity of the transactivating activity of substances and samples. The specific enzyme activity is expressed in AhR-units, which are the β-galactosidase-activity normalised to the total protein-level. The data were evaluated according Jungbauer et al. [18].

The results of the β-galactosidase-assay of the AhR screen in microtiterplates were referenced to cell density (measured as the optical cell density at a wavelength of 600 nm). The calculation of the AhR-units is realised as follows:

$$AU = \left( \frac{OD_{405} * 1000}{OD_{600} * \text{ml of cell suspension added}} \right) * \left( \frac{1}{\Delta t} \right)$$

$OD_{405}$ and $OD_{600}$ are the optical densities at a wavelength of 405 nm respectively 600 nm and $\Delta t$ is the incubation time at 37°C in minutes.

For evaluation the AhR-units are plotted against the concentration (logarithmic scaling). The resulting curve is fitted using a logistic dose response function. The calculation and fitting was performed with Table Curve 2D software (Jandel Scientific).

The equivalent concentrations of the extracts and samples were calculated from the logistic dose-response curve of β-naphtoflavone (Figure 8) and expressed as nmol β-naphtoflavone per g sample or volume of sample.

Example 5: Competitive Ligand Binding Assay

Affinity of preparations to estrogen receptor α and β(ER α and β) was determined by competitive radioligand binding assays described by Freyberger and Ahr [32]. Dilutions of the samples were combined with 50 µl 4.8 nM [$^3$H]-estradiol and 50 µl estrogen receptor solution (8 nM) and incubated for 16-18 h. In parallel non-specific binding was determined by adding an excess of unlabeled estradiol, which have a binding affinity to the corresponding receptors similar to the radioactivity of the labeled ligands. As a carrier protein albumin was added to the incubation buffer in a concentration of 3 mg/ml. The unbound radiolabeled ligand was removed by incubation with 100 µl dextran-coated charcoal for 15 min at 4 °C. Samples were centrifuged (6000 x g for 10 min at 4 °C), aliquots of the supernatant (100 µl) were added to scintillation cocktail (3 ml) and counted for 1 min. All determinations were carried out in duplicates. Counter efficiency was measured in parallel to each assay by measuring a known amount of [$^3$H]-estradiol.

**Example 6: Solid phase extraction**

Solid phase extraction (SPE) is a sample preparation technique based on the separation mechanisms of liquid chromatography. It is useful for concentration of liquid samples. The procedure was accomplished with reversed phase sorbent C18 columns to enrich hydrophobic compounds. The processing comprises conditioning of the column, sample preparation and addition, washing of the column and the elution of the sample with an appropriate eluting solvent.

The column conditioning was performed with hexane and acetone, in each case with 1 column volume and 2 column volumes acescent water (destillated water was acidified with $H_2SO_4$ to a pH of 3.5). The sorbent bed has to be kept wet during conditioning.

The samples were centrifuged and filtrated. Then the pH was adjusted to 3.5 with $H_2SO_4$ and 5% (v/v) acetone was added. The sample was applicated under vacuum conditions. The column was dried in a nitrogen stream. Afterwards the bound components were eluted with 2-3 columns volumes acetone. The eluate was evaporated at 38°C, the remainder dried in a nitrogen stream and dissolved in DMSO. The used amount of DMSO depends on the designated degree of enrichment. For a 1000fold concentration the remainder of a sample with a starting volume of 1 1 was dissolved in 1 ml DMSO.

It was also tested if an enrichment of the hydrophobic compounds could also be established with ethanol. Except for the solvents used, the implementation of the SPE was conducted as described above. 3 columns were parallel loaded with the same sauerkraut juice. The solvents used for column conditioning, elution and dissolving of the dried remainder, were variegated. SPE with Columns 1 was done classical with acetone/hexane conditioning, acetone addition

to the sample, elution with acetone and dissolving of the remainder in DMSO. With column 2 all steps were implemented with ethanol. Column 3 was like column 2, but the dissolving step was done with DMSO. The eluate was collected in 1 ml portions (column 1: 6x1 ml and columns 2 and 3: 10x1 ml) and separated processed. The fractions were analysed with the yeast AhR-test (Figure 11). Thus it appears, that the enrichment of the hydrophobic and active compounds of sauerkraut juice, also works with ethanol, even better. Most of the active compounds were eluted at the beginning (first two fractions). Therefore they were tested in dilutions (incorporated in the evaluation), while the other fractions were analysed undiluted. Recapitulating enrichment about a factor of 100 to 500 is possible.

**Example 7: RP-HPLC conditions**

The reversed-phase HPLC system for the analytical HPLC-analysis consisted of an Agilent 1100 series and was connected to a Luna C18(2)-column (Phenomenex 100x4.6 mm , particle diameter of 3 μm). Eluent A was 5% acetonitrile (AcCN)+0.1%TFA in water and eluent B was AcCN supplemented with 0.1% TFA. The flow rate was held constant at 0.5 ml/min. The following step gradient was used: 5 min 0%B, 25 min 0-17.5%B, 25 min 17.5-50%B, 10 min 50%B and up to 90%B for regeneration. 20 μl of the samples were injected and fractions were collected every minute or every 2 minutes, respectively. The solvent was evaporated in vacuum and the dried remainder dissolved in 100 μl DMSO. This solution was used to determinate the AhR-activity in the yeast assay. The preparative RP-HPLC was made analogue to the analytical HPLC. The Pharmacia FPLC System (Pharmacia Biotech, Piscataway, NJ) consisted of a Liquid Chromatography Controller LCC-500 Plus, 2 pumps (Pump P-3500) with a UV fixed wavelength detector (Monitor UV-M) and a REC 112 Dual-Channel Chart Recorder and was connected to a Luna C18(2)-column (particle diameter of 15 μm, Phenomenex 250x21.20 mm). The conditions were retained like for the analytical HPLC with a flow rate of 26.5 ml/min (according to ~0.3 column volume per minute for both HPLC columns). A sample volume of about 4 ml was injected and the fractions were collected every minute, and close-to the most active fractions (minute 50-56) in a time period of 15 sec. The solvent of the fractions was evaporated with the rotavapor and after a washing step with 10 ml AcCN evaporated again. The remainder was dissolved in 10 ml AcCN, aliquots of 1 ml portioned in 10 centrifuge tubes (Eppendorf, 1.5 ml) and the solvent evaporated with the speed vac. The dried remainders were dissolved in 100 μl AcCN and yoked together in a centrifuge tube (tare weight noted). The resulting 1 ml was evaporated and the remainder was weighted.

**Example 8: Transactivational potential of pure compounds**

With the optimised yeast AhR assays an assortment of pure compounds (dissolved in DMSO) was tested. The amount of expressed β-galactosidase, the product of the lacZ gene, correlates to the transactivational activity of the test substance. In Table 1 the results are summarised. In case the transactivation results could be fitted by a logistic dose response curve, the potencies are given.

Table 1: Results of the screening on pure compounds.

| Substance | Potency (mol/l) | Assay |
|---|---|---|
| Apigenin | active[c] | *,# |
| Biochanin A | $5.99\cdot10^{-7}$ $1.25\cdot10^{-7}$ | * # |
| Caffeic acid | no activity | * |
| Catechin | no activity | * |
| Coumestrol | $2.09\cdot10^{-6}$ | * |
| | $1.04\cdot10^{-6}$ | # |
| Curcumin | no activity | * |
| Daidzein | no activity | * |
| Diindolylmethane | $1.14\cdot10^{-6}$ | # |
| Diosmin | no activity | * |
| Epicatechin | no activity | * |
| Equol | active[c] | *,# |
| 17-β-Estradiol | no activity | * |
| Ferulic acid | no activity | * |
| Formononetin | $9.23\cdot10^{-7}$ | * |
| | $2.53\cdot10^{-7}$ | # |
| Gallic acid | no activity | * |

(continued)

| Substance | Potency (mol/l) | Assay |
|---|---|---|
| Genistein | no activity | * |
| Indole-3-carbinol | $7.00 \cdot 10^{-6}$ | # |
| ITE | $4.32 \cdot 10^{-9}$ | * |
| | $7.82 \cdot 10^{-10}$ | # |
| β-Naphtoflavone | $1.0 \cdot 10^{-8}$ | * |
| | $2.8 \cdot 10^{-7}$ | # |
| Naringenin | active[c] | *,# |
| Phloretin | no activity | * |
| Phtalic acid dibutyl ester | no activity | * |
| Progesteron | no activity | * |
| Quercetin | no activity | * |
| Resveratrol | no activity | * |
| β-Sitosterol | no activity | * |
| Tangeritin | no activity | * |

* - in plastic tubes
\# - in microtiterplate
c - no saturated ligand-receptor-binding achieved

As comparative references the microtiterplate results are used. Compounds with a lower molecular weight, such as caffeic acid, ferulic acid or gallic acid (molecular mass about 170-194 g/mol) showed no activity. Most of the active compounds have a molecular weight in the range of 270-284 g/mol. Tested hormones, such as 17-β-estradiol or progesterone failed also in transactivating the AhR, as well as the synthetic hormone-like substance diethylstilbestrol or the phytosterole β-sitosterol. Some compounds, which were mentioned in the literature as oestrogenic substances, such as daidzein, genistein, tangeretin, resveratrol and quercetin, revealed no activity in the yeast AhR screen assay.

In the category of natural occurring compounds two categories became apparent: the isoflavones and indole compounds. Although several flavonoids were mentioned as AhR agonists or antagonists isoflavones are not associated with this receptor. Ashida [5] analysed the isoflavones daidzein and genistein for their suppressive effect on dioxin toxicity, but failed. The isoflavones biochanin A and formononetin have not been tested by transactivating AhR tests before. They are well-known as phytoestrogens and main components of red clover, a plant which is used for easing of menopausal disorders [16]. Sakakibara et al. [17] detected high amounts of formononetin (50 μg/l) and equol (210 μg/l) in milk from goats fed with clover. These amounts conform to $1.9 \cdot 10^{-7}$ and $8.7 \cdot 10^{-7}$ mol/l, concentrations that are close to EC50 values that were determined with oestrogenic in-vitro tests [18, 19, 20]. Interestingly potencies about $1.2 \cdot 10^{-7}$ to $9.2 \cdot 10^{-7}$ mol/l for biochanin A and formononetin could also be detected with the yeast AhR assay (Figure 2A). The isoflavones daidzein and genistein showed no transcriptional activity (Figure 2A). Also the second category, the indole compounds, exhibit notable potencies (Figure 2B). Diindolylmethane showed a potency of $1.1 \cdot 10^{-6}$ mol/l and indole-3-carbinol $7.0 \cdot 10^{-6}$ mol/l. These compounds are compounds of cruciferous plants, such as broccoli, cabbage and cauliflower. Indole-3-carbinol is generated by enzymatic degradation of glucobrassicin, a main glucosinolate of cabbage (31.95 μmol/100 g of fresh weight [21] or 49 μmol/100 g dry weight [22]). The content of indole-3-carbinol in fermented cabbage stored for 2-17 weeks is very stable in a range of 4.7 to 5.1% of the glucobrassicin, that is contained in the fermented cabbage [21]. Indole-3-carbinol is the precursor of diindolylmethane, which is the major acidcatalysed metabolite formed in the gut. These indole compounds are proved to have antitumorigenic and antioestrogenic properties [23].

Naringenin, apigenin and equol could not achieve the saturation of receptor ligand-binding, but apparently a transactivation took place (Figure 2). All data were obtained with the yeast assay in plastic tubes and normalised to the maximum of the logistic dose response curves of β-naphtoflavone.

**Example 9: Activation by extracts of food, beverages and supplements**

Extracts in DMSO of diverse food, beverages, herbs, spices and supplements were screened for their transactivating potential of AhR. The results were related to β-naphtoflavone and expressed as equivalent concentrations of the reference (nmol b-naphtoflavone/g sample or nmol β-naphtoflavone/l sample). The results are listed in Table 2.

Table 2: AhR-Activating potential of diverse food and herb extracts.

| **Extract in DMSO** | **equivalent Concentration** (nmol β-Naphtoflavone/g sample) | **Assay** |
|---|---|---|
| Anis seed | not detectable | * |
| Arrow root | 11.43 | # |
| Barbecue residues | 121.05 | * |
| Blackhaw | not detectable | * |
| Bitter melon | 16.21 | # |
| Broccoli | 28.52 | * |
| Bugleweed | not detectable | * |
| Burdock root | 29.66 | * |
| Cabbage (white) | 6.70 | # |
| Carob | not detectable | * |
| Cimicifuga (black cohosh): tablets obtained from | | |
| Bionorica (Klimadynon) | 178.82 | * |
| tablets obtained from Riemser Arzneimittel AG (Jinda) | 25.48 | * |
| tablets obtained from Schaper&Brümmer (Remifemin) | 41.82 | * |
| Cinnamon | not detectable | * |
| Clove | 165.16 | # |
| Coffee | 133.57 | * |
| Fennel | not detectable | * |
| Fenugreek seed | not detectable | * |
| Garden cress | 21.28 | # |
| Garlic (deep-frozen) | not detectable | * |
| Garlic (powdered) | not detectable | * |
| Kale | 13.84 | # |
| Kuzu | 2.83 | # |
| Lady's Mantle | not detectable | * |
| Maca | not detectable | * |
| Mallow | not detectable | * |
| Milfoil | not detectable | * |
| Nutmeg | not detectable | * |
| Onion (powdered) | not detectable | * |
| Onion (red) | 19.80 | # |
| Oregano | 57.90 | # |
| Orris root | 13.57 | * |
| Parsley | 23.51 | # |
| Pepper (black) | 11734.26 | * |
| Perga | not detectable | * |
| Pomegranate | not detectable | * |
| Potentilla | not detectable | * |
| Pueraria | 1281.03 | * |
| Sage (tealeaves) | 257.23 | * |
| Shepherd's Purse | not detectable | * |
| Tansy | not detectable | * |
| White Dead Nettle | not detectable | * |
| Wild Yam | 397.34 | * |

* in plastic tubes
# in microtiterplate

The results of the activational potential of the 100 fold enriched juices are demonstrated in Figure 3A. Sauerkraut showed an extremely high potential. This may be due to some ingredients, that are found in fermented cabbage, such as indole-3-carbinol and diindolylmethane, that are ligands of the AhR too. The AhR activating effect of sauerkraut could also be detected without sample concentration (Figure 3B). But juices of different producers showed differing results. Some other juices that showed a slightly activation are from celery, beetroot, stinging-nettle, dandelion.

Given that a lot of compounds, that are known to have oestrogenic activity, like the isoflavones and coumestrol, also activate the AhR pathway, a selection of soy, red clover and black cohosh supplements, that contain as main part isoflavones like daidzein, genistein, glycitein, formononetin and biochanin A, were tested.

According to the fact that red clover contains totalling 4.5 to 4.9 mg biochanin A and formononetin/g red clover, whereas daidzein and genistein amount to about 0.2 mg/g [24], the preparations based on red clover were the strongest activators of the AhR. Whereas the preparations based on soy excited the weakest activation, due to the fact that soy contains as major isoflavones daidzein, genistein and glycitein and only in traces biochanin A and formononetin. Of the preparations that are made of black cohosh extract, the content of isoflavones is not remarked on the package inserts. The specification on the package of the preparations made of black cohosh extract always refers to the extract amount of black cohosh rootstock. Black cohosh contains formononetin in a range of 3.1-3.5 μg/g dry weight [25]. Most of the tested preparations are as potent ligands of the AhR, as it can be presumed from their composition of isoflavones.

**Example 10: Analysis of sauerkraut juices**

Different sauerkraut juices were tested on their ability to activate aryl hydrocarbon receptor in the yeast test system. All of them showed a transactivation potential, like the $EC_{50}$ value of the standard compound β-naphtoflavone of about $1*10^{-8}$ M. The sauerkraut juice "Ja! Naturlich®" was used for further experiments.

To enrich the hydrophobic compounds of the juice a solid phase extraction (SPE) via reversed phase C 18 columns was conducted. The success of the SPE was measured by the AhR activity after each purification step. There was only a minimal reduction after the centrifugation and filtration steps and the flow through of the SPE column showed only a low AhR activity (Figure 9). Additionally the AhR activity of the eluate and dilutions from this concentrate was measured. A linear relation could be detected and the activity of a 1:100 dilution from the 100 fold sauerkraut concentrate was in the same range like the raw juice (Figure 10).

100 μl of the hundredfold concentrated sauerkraut juice was analysed with an analytical reversed phase C 18 (2) column (Figure 4). To separate the different compounds and to determine the AhR active ones fractions in 1 minute interval were collected after separation on the analytical C 18 (2) column. The fractions were dried in the speed vac, dissolved in DMSO and then AhR activity was measured. The same procedure was done with 100 μl DMSO as blank. The fractions with the more hydrophobic compounds showed the higher AhR activity (minute 45 to 55). The highest activity was measured in the fraction from minute 52 to 54, this was also equivalent with the highest peak in the chromatogram (Figure 5).

Activity of the fractions was determined as described above. Fractions with highest activity were between minutes 40-60 (fractions 20-30) (Figure 5). In another run fractions after minute 50 of the sauerkraut HPLC were collected in 0.5 min time intervals measured by the yeast assay (figure 8). Highest activity was measured between minutes 52-53.5 (fractions 5-7).

In order to determine the retention times of indole-3-carbinol and diindolylmethan, these compounds were applied to a similar reversed phase HPLC C 18 column under the same conditions. Indole-3-carbinol eluates at minute 70 and diinolylmethane eluates at minute 67. The grey arrow indicates the time where the highest AhR activity was measured in the sauerkraut HPLC (minute 53), which can therefore not be related to these compounds (Figure 7).

**Example 11: Chromatographic behaviour of indole compounds**

To study the chromatographic behaviour of AhR ligands in relation to new purified compound ("X"), DIM (3,3'-dindolylmethane), ICZ (indolo-[3,2-b]carbazole), I3C (indole-3-carbinol) and tryptanthrin were tested on a analytical C18 (2) HPLC column under the same chromatographic conditions (Figure 12A). Only tryptanthrin had a similar retention time, ICZ, I3C and DIM are more hydrophobic. Then all 5 compounds were mixed and separated within one run. Tryptanthrin and compound "X" are very similar in their hydrophobic properties, but they could also be separated from the mixture (Figure 12B).

**Example 12: pH and thermostability of AhR stimulating potential of sauerkraut juices**

Raw sauerkraut juice was adjusted to a pH of 7.0 and 8.0 with NaOH, and the activity of these juices and supernatants (small amounts of a precipitate occurred) was tested with the AhR yeast assay. A change of pH does not seem to affect the AhR activity of the juice (Figure 13).

To test the effect of increased temperature, sauerkraut juice was tempered for 4, alternatively 6 hours at 60°C, and afterwards tested with the AhR yeast assay (Figure 14).

No significant change was observed with a juice with normal pH of 3.9, but a juice of a pH of 7.0, that was tempered with an increased temperature exhibits a higher activity. It is possible that some combustion products were formed, and that the higher activity is due to a change of compound composition.

**Example 13: Isolation of compound "X" (1-(2-furanyl)2-(3-indolyl)ethanone)**

Before isolation of the compound for NMR-analysis, a preliminary isolation of the compound using the same preparative HPLC-column was conducted. This was tested on AhR-activity with the yeast assay (Figure 15). The equivalent concentration of the compound adds up to 79 μmol β-naphtoflavone per gram sample.

The analytical separation procedure was scaled-up to a preparative method using a ~80 ml preparative HPLC-column, to identify the compound eluting as highest peak (Figure 16). Therefore a larger amount of a 100fold concentrate was produced starting from 4 l of natural sauerkraut juice. This eluate was further purified by preparative RP-HPLC and the fractions were collected. The fractions of each chromatographic run with the highest peak at a retention time of about 52 min were pooled and subjected to NMR-analysis. In parallel the purification was controlled by an analytical RP-HPLC-analysis (Figure 17).

The NMR-identified compound "X" has an indole structure (Figure 19),

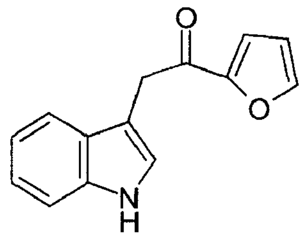

(formula 1)

with an empirical formula of $C_{14}H_{11}NO_2$ and thus a molecular weight of 225.28 g/mol.

**Example 14: Estrogen receptor ligand binding assay**

The 100 fold concentrated sauerkraut juice was tested for the ability to bind to the estrogen receptor α and β. The highest sample concentration showed a significant effect in the competitive ligand binding assay for the ER α and β (Figure 18).

No transcriptional activity on the ER α of the 100fold concentrated sauerkraut juice could be detected in the yeast estrogen assay (performed according to Klinge et al. [33]).

In an in vivo study with rats, the 200fold concentrated sauerkraut juice, exhibited low anti-estrogenic properties.

**Example 15: Derivatisation of 1-(2-furanyl)2-(3-indolyl)ethanone**

Introduction of methoxy group at position 1 is obtained by anodic methoxylation of 1-(2-furanyl)2-(3-indolyl) ethanone in presence of alkaline methanol. Reaction products are separated by reversed phase HPLC using a C18 column with acetonitrile as a mobile phase modifier.

Anodic fluorination (1 mmol) was carried out with platinum plate electrodes (2 x 2 $cm^2$) in MeCN (10 mL) containing a fluoride salt (1 M), using an undivided cell at room temperature. Constant current (10 mA $cm^{-2}$) was applied. The conversion of a starting material was monitored by TLC. After the charge was passed (4 F $mol^{-1}$) until the starting material was consumed, the electrolytic solution was passed through a short column of silica gel eluting with $CHCl_3$ to remove the fluoride salt. The eluent was evaporated under vacuum. The reaction products were separated by reversed phase HPLC using acetronitrile as a mobile phase modifier.

**Example 16: NMR data assignment of the HPLC extract of 1-(2-furanyl)2-(3-indolyl)ethanone recorded at 300 K in DMSO-$_{d6}$**

NMR data assignment of the HPLC extract recorded at 300 K in DMSO-$_{d6}$

| | Indole-3→ | | | →$CH_2$-CO- 2- Furyl | | |
|---|---|---|---|---|---|---|
| | $^{1}$H | | $^{13}$C | $^{1}$H | | $^{13}$C |
| Atom position | Chemical Shift (ppm) | *J* (Hz) | Chemical Shift (ppm) | Chemical Shift | *J* | Chemical Shift |
| 1 | 10.94 | <1.0 | - | - | - | - |
| 2 | 7.27 | <1.0 | 124.32 | - | - | 151.46 |
| 3 | - | - | 107.20 | 7.60 | 3.6 | 118.89 |
| 4 | 7.51 | 7.8 | 118.48* | 6.71 | 3.6, <1.0 | 112.44 |
| 5 | 6.97 | 7.1,7.8 | 118.57* | 7.98 | <1.0 | 147.72 |
| 6 | 7.06 | 7.1,7.9 | 121.01 | | | |
| 7 | 7.34 | 7.9 | 111.37 | | | |
| 8 | - | - | 136.05 | | | |
| 9 | - | - | 127.15 | | | |

Other signals: 4.21 (CH2) and 35.07 ppm (CH2) and 186.10 ppm (CO) as well as signals between 62.6-69.25 and in the aliphatic range

* assignments may be reversed

**Example 17: NMR connectivities of the HPLC extract of 1-(2-furanyl)2-(3-indolyl)ethanone recorded at 300 K in DMSO-$_{d6}$**

NMR connectivities of the HPLC extract recorded at 300 K in DMSO-$_{d6}$

| | Indole.3→ | | | | →$CH_2$-CO- 2- Furyl | | | |
|---|---|---|---|---|---|---|---|---|
| | $^{1}$H | NOESY | $^{13}$C | HMBC | $^{1}$H | NOESY | $^{13}$C | HMBC |
| Atom position | Chemical Shift | | Chemical Shift | | Chemical Shift | | | Chemical Shift |
| 1 | 10.94 | | - | - | - | - | | |
| 2 | 7.27 | 4.21 | 124.32 | 107.20, 136.05, 127.18 | - | - | 151.46 | - |
| 3 | - | - | 107.20 | - | 7.60 | 6.71, 7.27 | 118.89 | 112.44, 147.72, 151.46 |
| 4 | 7.51 | 6.97, 4.21 | 118.48* | 121.01, 136.05, 107.20 | 6.71 | 7.98,7.60 | 112.44 | 118.89, 151.46 |
| 5 | 6.97 | 7.51 | 118.57* | 111.37, 127.18 | 7.98 | 6.71 | 147.72 | 118.89, 131.46 |
| 6 | 7.06 | 7.34 | 121.01 | 118.48, 136.05 | | | | |
| 7 | 7.34 | 7.06 | 111.37 | 118.57, 127.18 | | | | |
| 8 | - | - | 136.05 | | | | | |

(continued)

| | Indole.3→ | | | | →$CH_2$-CO- 2- Furyl | | | |
|---|---|---|---|---|---|---|---|---|
| | $^{1}H$ | NOESY | $^{13}C$ | HMBC | $^{1}H$ | NOESY | $^{13}C$ | HMBC |
| Atom position | Chemical Shift | | Chemical Shift | | Chemical Shift | | | Chemical Shift |
| 9 | - | - | 127.15 | | | | 35.07 | 107.60, 127.15 186.10, 151.46 |
| Other signals: 4.21 (CH2) and 35.07 ppm (CH2) and 186.10 ppm (CO) as well as signals between 62.6-69.25 and in the aliphatic range<br>* assignments may be reversed | | | | | | | | |

**References :**

1. Burbach et al.,Proc Natl Acad Sci U S A, 89 (1992) 8185-8189.
2. Amakura et al., Biol Pharm Bull, 26 (2003) 1754-1760.
3. Amakura et al., Biol Pharm Bull, 25 (2002) 272-274.
4. Jeuken et al., J Agric Food Chem, 51 (2003) 5478-5487.
5. Ashida et al., Biofactors, 12 (2000) 201-206.
6. Zhang et al., Environ Health Perspect, 111 (2003) 1877-1882.
7. Zhou et al., Mol Pharmacol, 63 (2003) 915-924.
8. Hahn et al., Mar Biotechnol (NY), 3 (2001) S224-238.
9. Mimura et al., Biochim Biophys Acta, 1619 (2003) 263-268.
10. Petrulis et al., J Biochem Mol Toxicol, 14 (2000) 73-81.
11. Swanson et al., J Biol Chem, 270 (1995) 26292-26302.
12. Denison et al., Toxicol, 43 (2003) 309-334.
13. Miller III et al., Toxicology and Applied Pharmacology, 160 (1999) 297-303.
14. Kippert et al., FEMS Microbiol Lett, 128 (1995) 201-206.
15. Adachi et al., Journal of Biological Chemistry, 276 (2001) 31475-31478.
16. Beck et al., J Steroid Biochem Mol Biol, 84 (2003) 259-268.
17. Sakakibara et al., Biofactors, 22 (2004) 237-239.
18. Jungbauer et al., Journal of Chromatography B: Analytical Technologies in the Biomedical and Life Sciences, 777 (2002) 167-178.
19. Kostelac et al., J Agric Food Chem, 51 (2003) 7632-7635.
20. Promberger et al., J Agric Food Chem, 49 (2001) 633-640.
21. Ciska et al., J Agric Food Chem, 52 (2004) 7938-7943.
22. Tolonen et al., J Agric Food Chem, 50 (2002) 6798-6803.
23. Chen et al., Carcinogenesis, 19 (1998) 1631-1639.
24. Krenn et al., J Chromatogr B Analyt Technol Biomed Life Sci, 777 (2002) 123-128.
25. Panossian et al., Phytochem Anal, 15 (2004) 100-108.
26. Forsythe et al., Mol Cell Biol, 16 (1996) 4604-4613.
27. Shams et al., Acad Sci U S A, 101 (2004) 9698-9703.
28. Pollenz et al., Mol Pharmacol, 56 (1999) 1127-1137.
29. Kozak et al., Dev Biol, 191 (1997) 297-305.
30. Kassie et al., Chem-Biol Interact 142 (2003) 285-296.
31. Kassie et al., Carcinogenesis 23(7) (2002) 1155-1161.
32. Freyberger and Ahr, Toxicology 195(2-3) (2004) 113-126.
33. Klinge et al., Journal of Agricultural and Food Chemistry 51(7) (2003) p. 1850.
34. Lysenkova et al., Russian Chemical Bulletin, Int. Ed., 50(7) (2001) 1309.
35. Lavrenov et al., Pharmaceutical Chemistry Journal 39(5) (2005) 251.

## Claims

1. A medicament comprising a chemical compound according to formula 1:

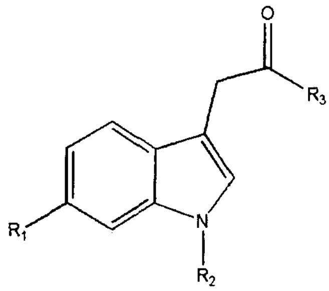

(formula 1)

wherein R1 is hydrogen, fluorine or methoxy, R2 is hydrogen, methyl, ethyl, propyl or fluorine and R3 is furanyl or pyranyl.

2. Medicament according to claim 1, **characterised in that** the chemical compound is 1-(2-furanyl)2-(3-indolyl)ethanone (formula 2) :

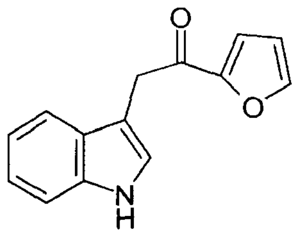

(formula 2).

3. Use of biochanin A, formononetin, a compound of formula 1 as defined in claim 1, preferably 1-(2-furanyl)2-(3-indolyl)ethanone, or mixtures thereof, for the production of a pharmaceutical or nutritional preparation for the activation of the aryl hydrocarbon receptor.

4. Use of biochanin A, formononetin, a compound of formula 1 as defined in claim 1, preferably 1-(2-furanyl)2-(3-indolyl)ethanone, or mixtures thereof, as aryl hydrocarbon receptor activator ex vivo.

5. Use according to claim 3, **characterised in that** biochanin A, formononetin, a compound of formula 1 as defined in claim 1, preferably 1-(2-furanyl)2-(3-indolyl)ethanone, are used for the production of a preparation for treatment of menopausal disorders, detoxification in smokers, abatement of hypoxia, anti-ageing, cancer treatment or cancer prevention, anti-inflammation and increased wound-healing.

6. Use according to claim 5, **characterised in that** the production of a preparation is for the treatment of cancer and the cancer is selected from liver cancer, lung cancer, colon cancer, colorectal cancer, breast cancer, prostate cancer, skin cancer, papilloma virus induced cancer, in particular cervical-vaginal cancer.

7. Use according to claim 3 or 4 for the production of cytochrome P450 or glutathione-S-transferase Ya.

8. Method for the isolation of a compound of claim 2, comprising a chromatographic purification of sauerkraut or sauerkraut juice.

9. Use of a plant extract or plant juice for the production of a pharmaceutical or nutritional preparation for the activation of the aryl hydrocarbon receptor, wherein the plant extract or juice comprises aryl hydrocarbon receptor activity.

**10.** Use according to claim 9, **characterised in that** the plant is selected from cruciferous vegetables.

**11.** Use according to claim 9 to 10, **characterised in that** the plant is a fermented plant, preferably fermented by a lactobacillus.

**12.** Use according to any one of claims 9 to 11, **characterised in that** the plant is selected from sauerkraut, pepper, black pepper, cress, clover, red clover, pueraria, or mixtures thereof, preferably from sauerkraut or cress.

**13.** Use according to any one of claims 9 to 12, **characterised in that** the plant comprises apigenin, biochanin A, coumestrol, diindolylmethane, equol, formononetin, indole-3-carbinol, 2-(1'H-indole-3'-carbonyl)-thiazole-4-carboxylic acid (ITE), naringenin, 1-(2-furanyl)2-(3-indolyl)ethanone, or a metabolic precursor thereof.

**14.** Use according to any one of claims 9 to 13, **characterised in that** the preparation is a dry preparation.

**15.** Use according to any one of claims 9 to 14, **characterised in that** the preparation is a concentrated extract or juice, preferably concentrated by a factor 2 to 500, preferably 5 to 400, more preferred 10 to 300, most preferred 80 to 150, relative to the original plant mass.

**16.** Use according to any one of claims 9 to 15, **characterised in that** the plant extract or juice has an aryl hydrocarbon receptor activating potential of at least 2000, preferably at least 3000, even more preferred at least 5000, equivalent nmol β-naphto-flavone/g sample.

**17.** Use according to any one of claim 9 to 16, **characterised in that** the preparation is prepared by the steps of

• obtaining an extraction or juice of the plant comprising an aryl hydrocarbon receptor ligand,
• optionally thickening of the extract or juice, preferably by vacuum evaporation, and/or solid phase extraction, preferably with ethanol,
• drying the extract or juice,
• packaging of the dry product.

**18.** Use according to claim 17, **characterised in that** the dry product is packaged into capsules or blister packs.

**19.** Use according to any one of claims 9 to 18 for the production of a preparation for treatment of menopausal disorders, detoxification in smokers, abatement of hypoxia, anti-ageing, cancer treatment or cancer prevention, anti-inflammation and increased wound-healing.

**20.** Use according to claim 19, **characterized in that** the production of a preparation is for the treatment of cancer and the cancer is selected from liver cancer, lung cancer, colon cancer, colorectal cancer, breast cancer, prostate cancer, skin cancer, papilloma virus induced cancer, in particular cervical-vaginal cancer.

**21.** Method for the activation of the aryl hydrocarbon receptor using a plant extract or plant juice according to any one of claims 9 to 20 or using biochanin A, formononetin or a compound of formula 1 as defined in claim 1, preferably 1-(2-furanyl)2-(3-indolyl)ethanone, as aryl hydrocarbon receptor activator.

Figure 1

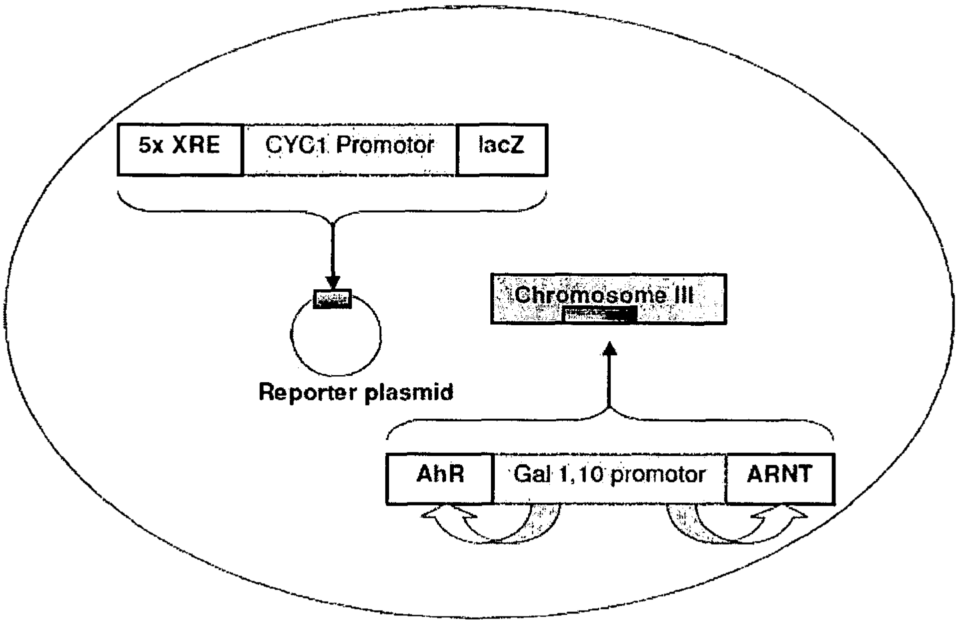


Figure 2A

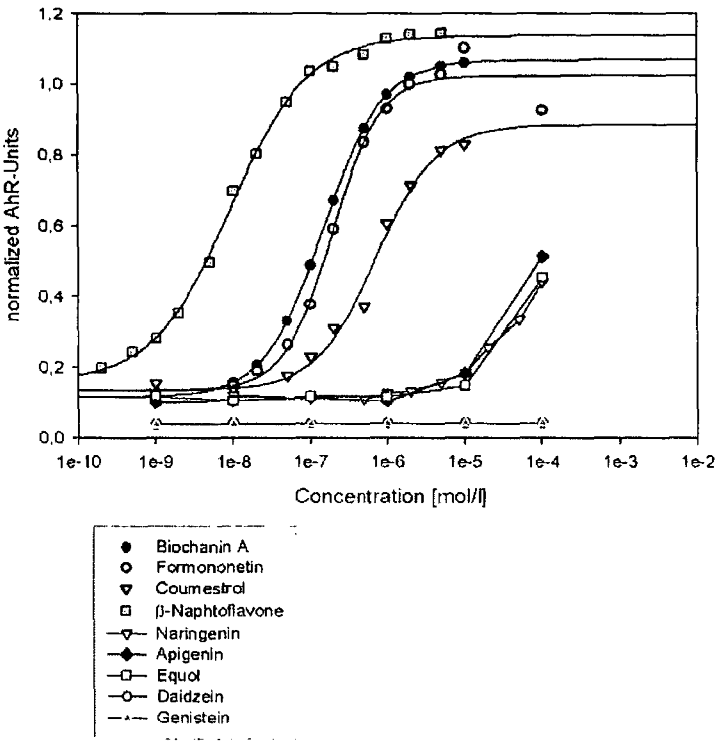

Figure 2B

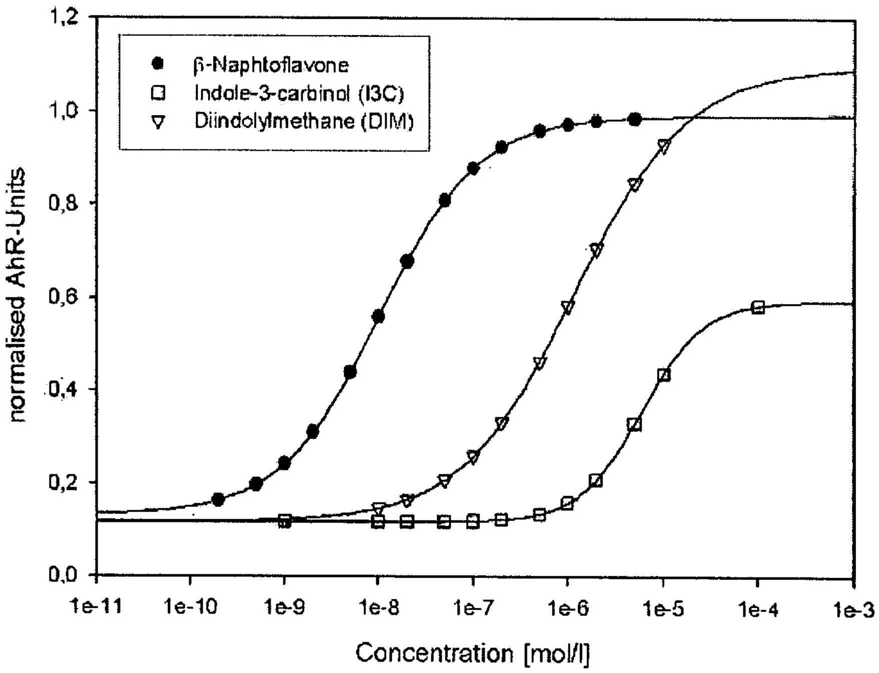


Figure 3A

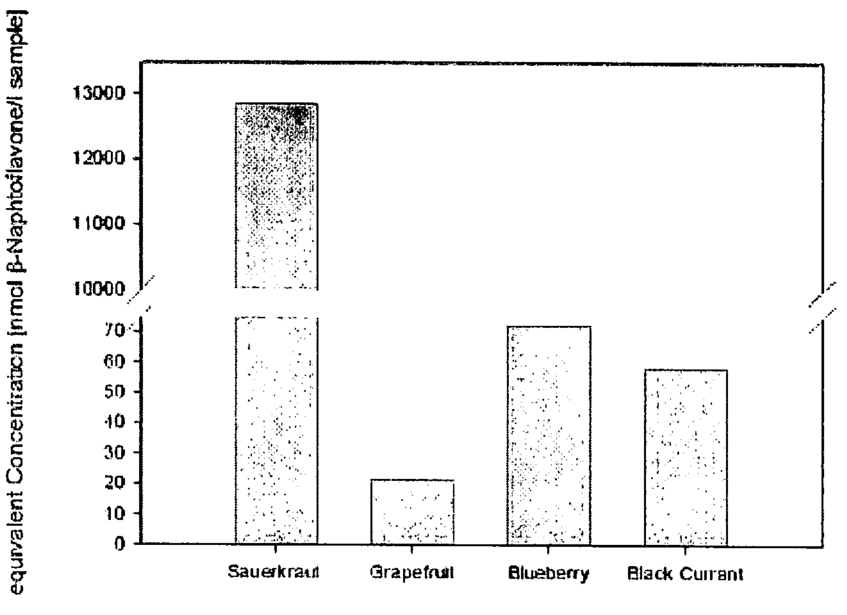

Figure 3B

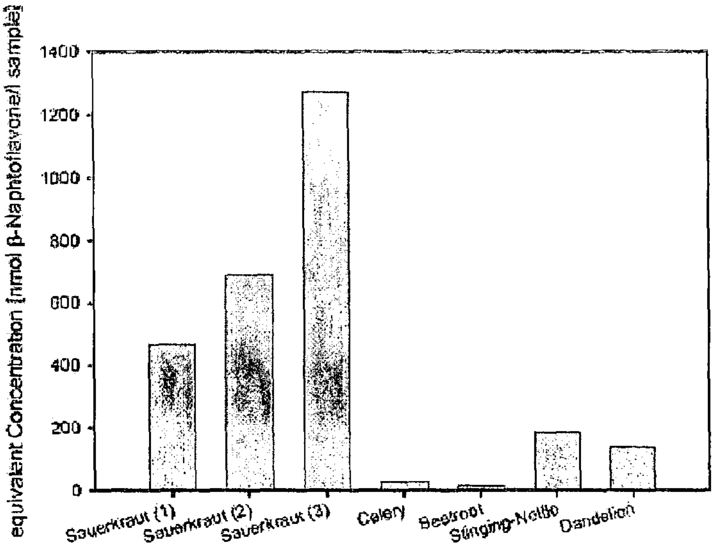


Figure 4

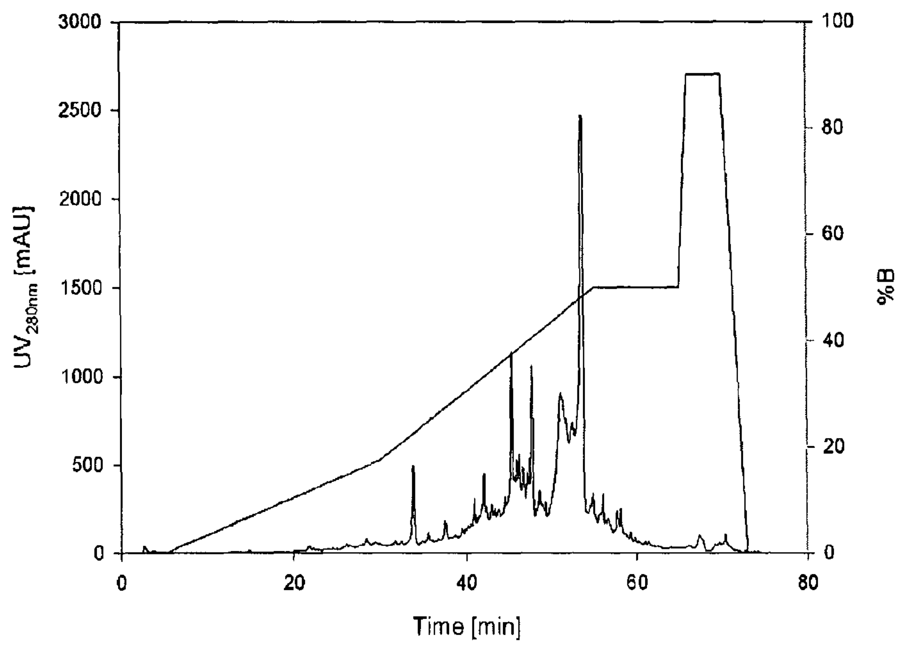

Figure 5

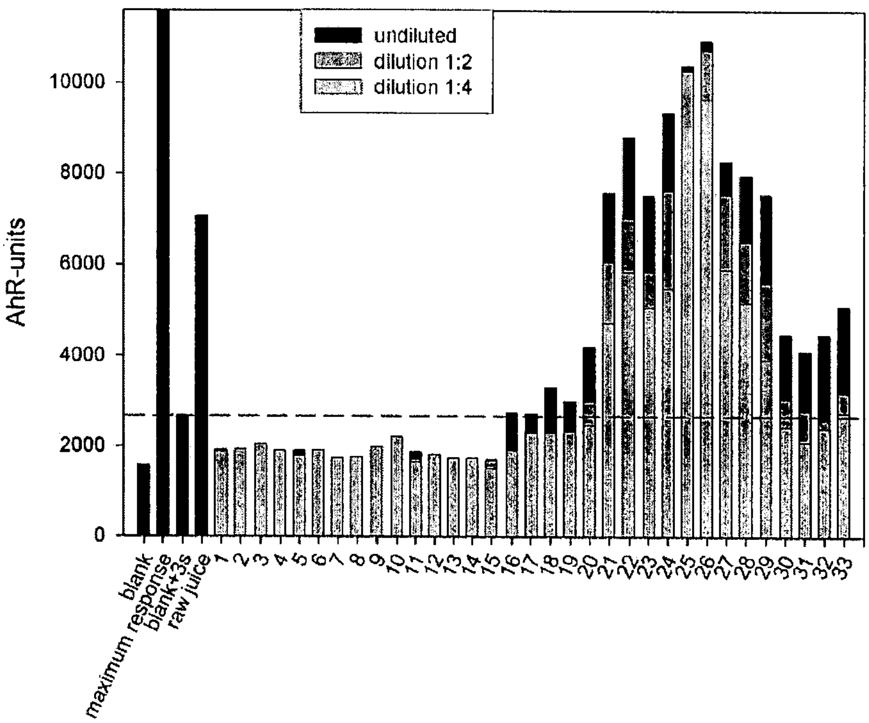


Figure 6

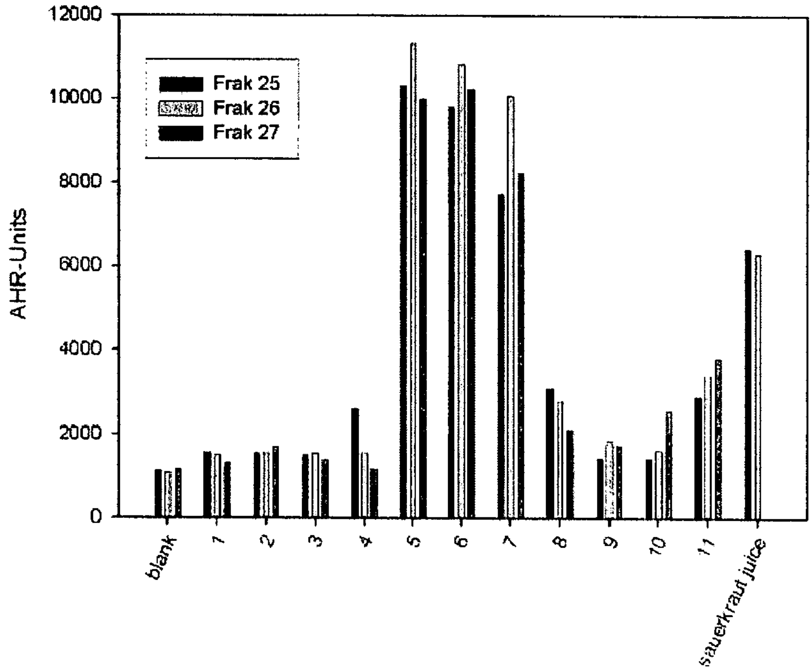

Figure 7

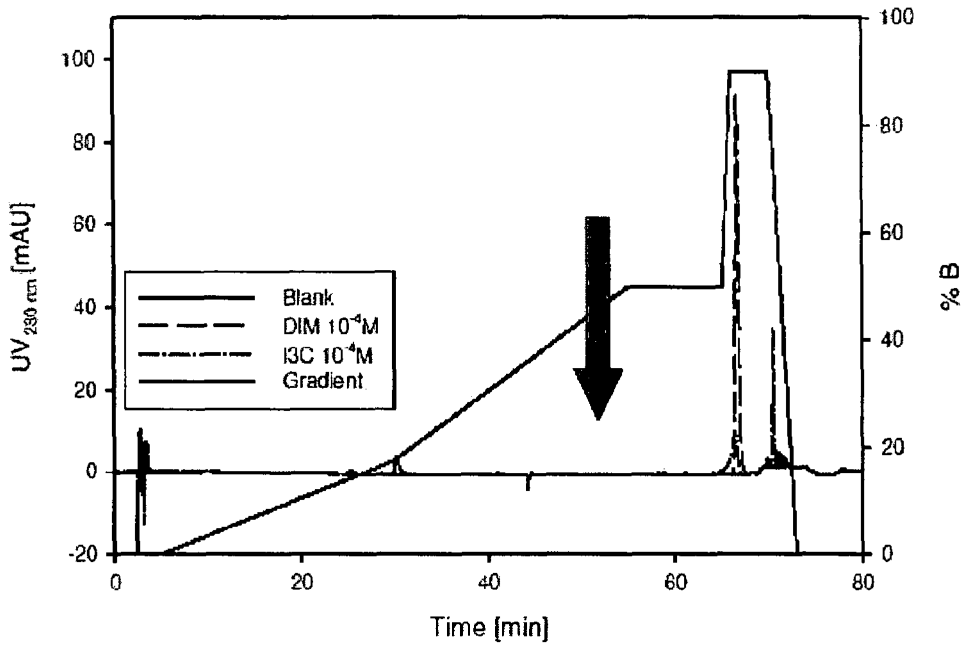


Figure 8

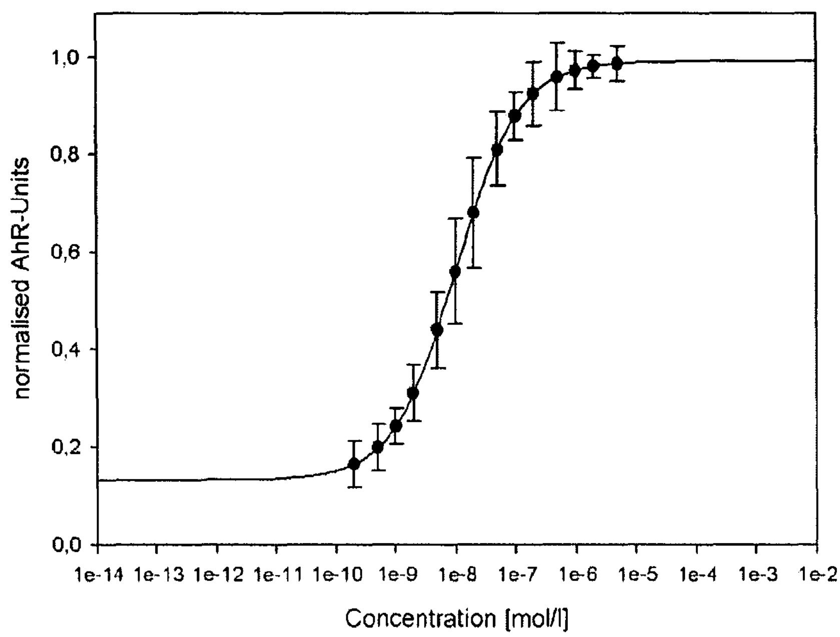

Figure 9

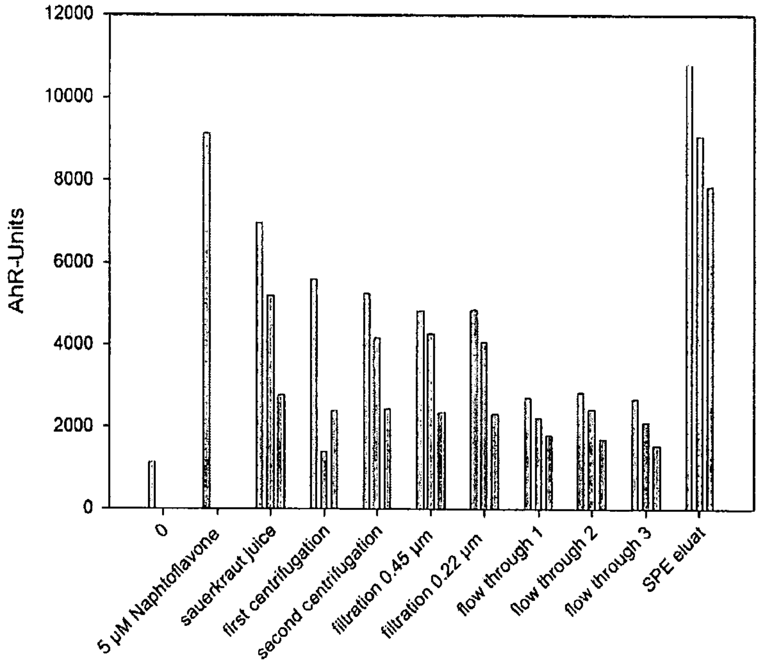


Figure 10

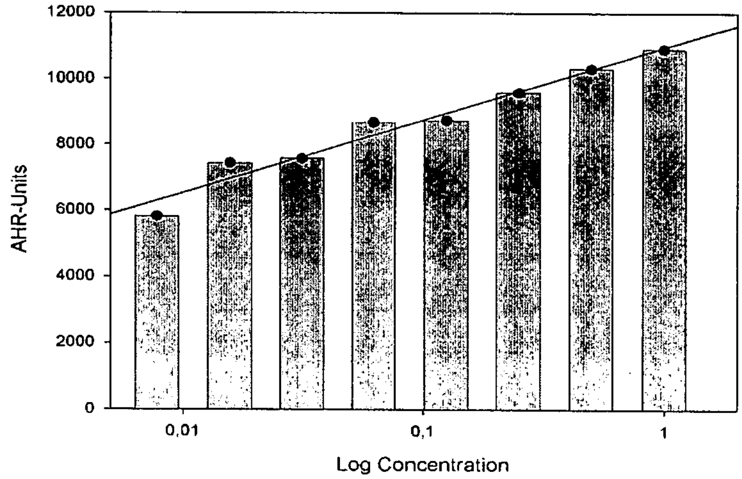

Figure 11

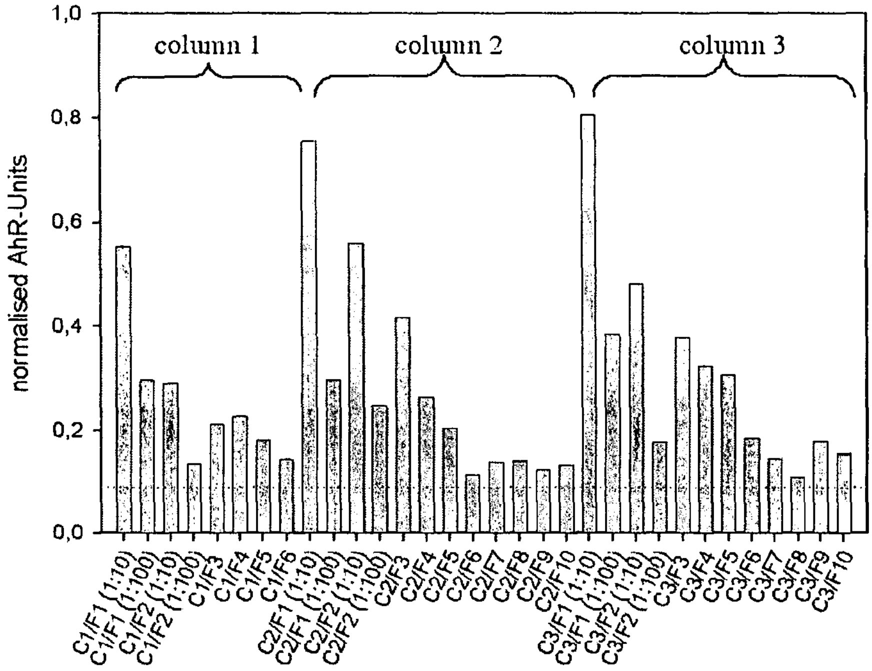


Figure 12A

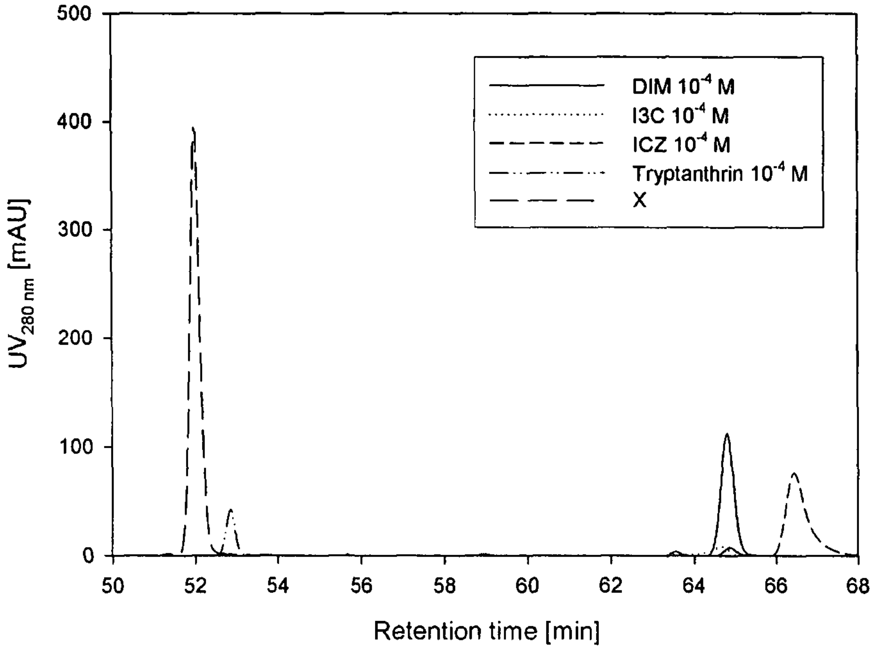

Figure 12B

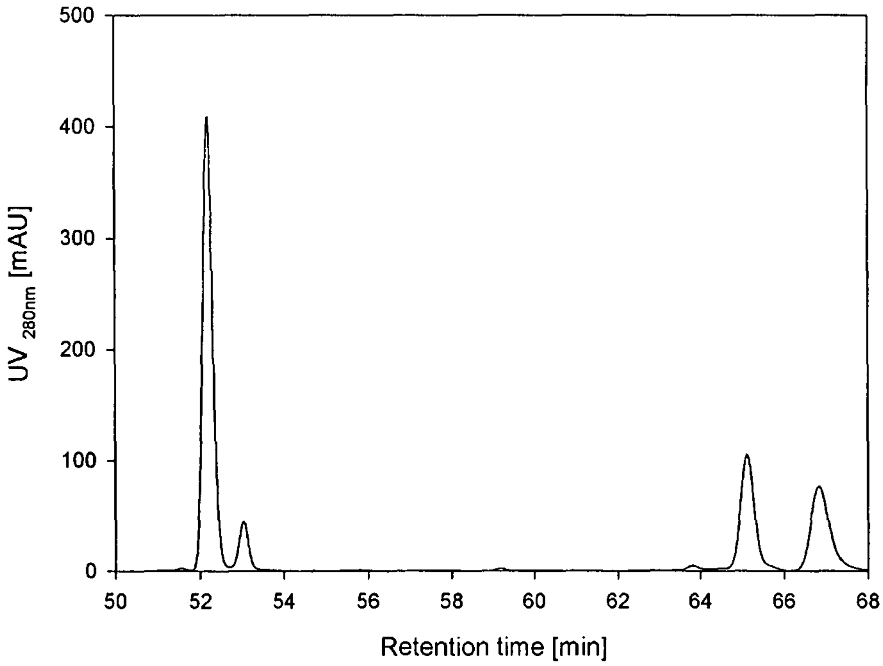


Figure 13

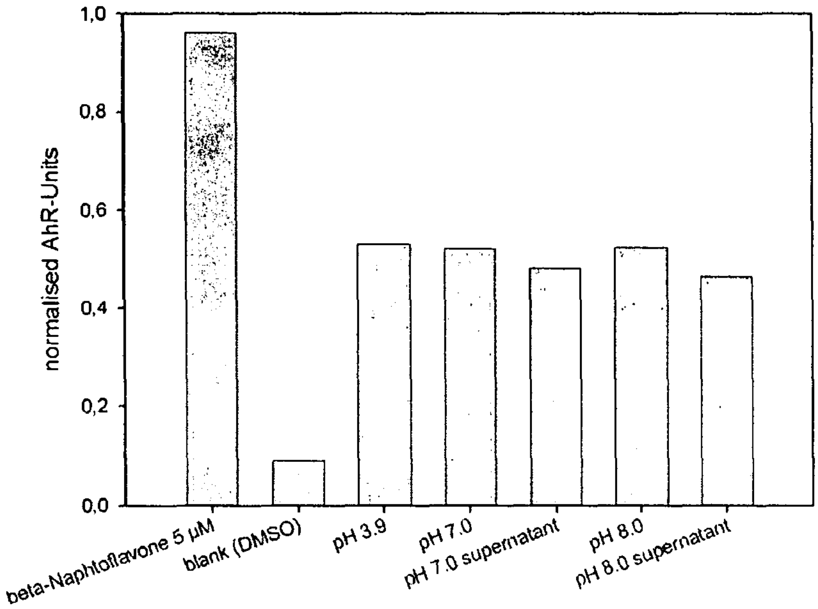

Figure 14

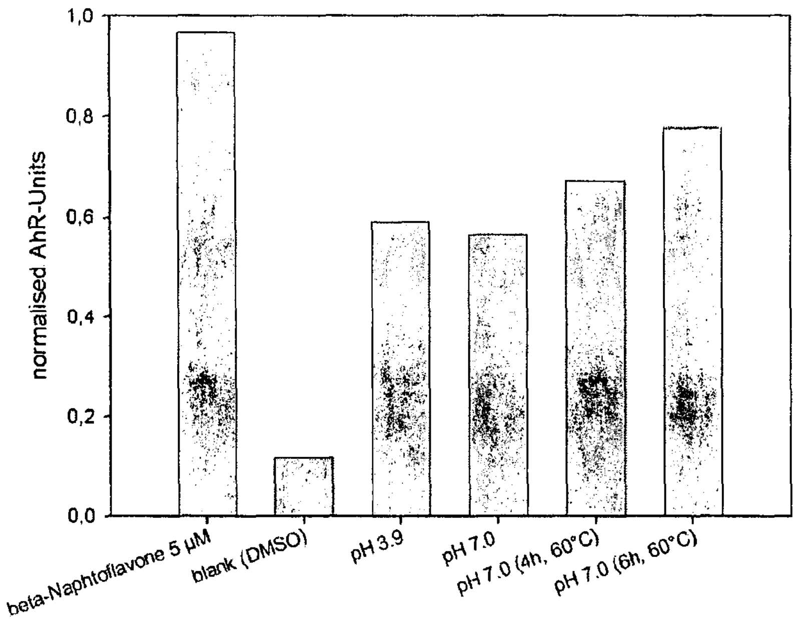


Figure 15

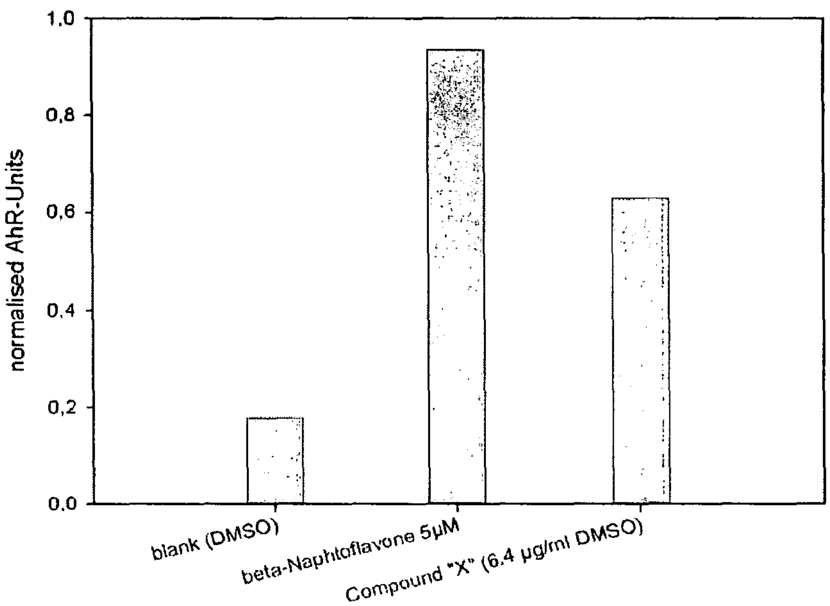

Figure 16

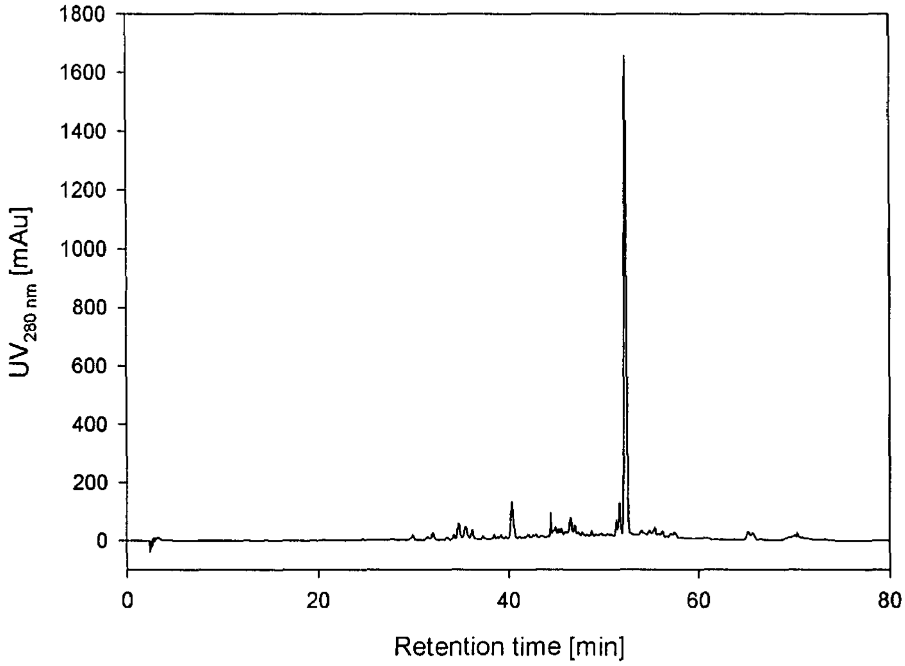


Figure 17

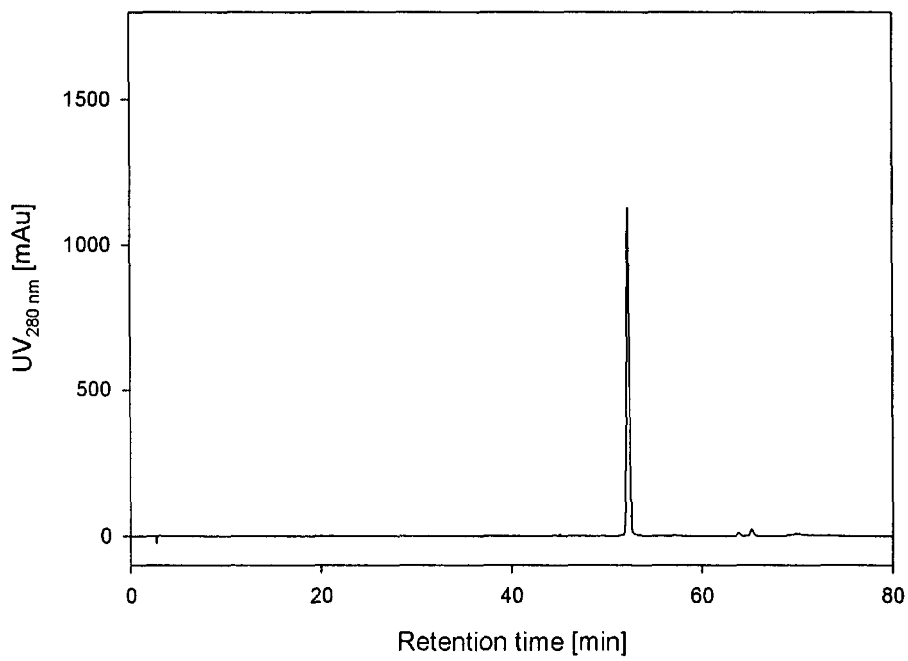

Figure 18

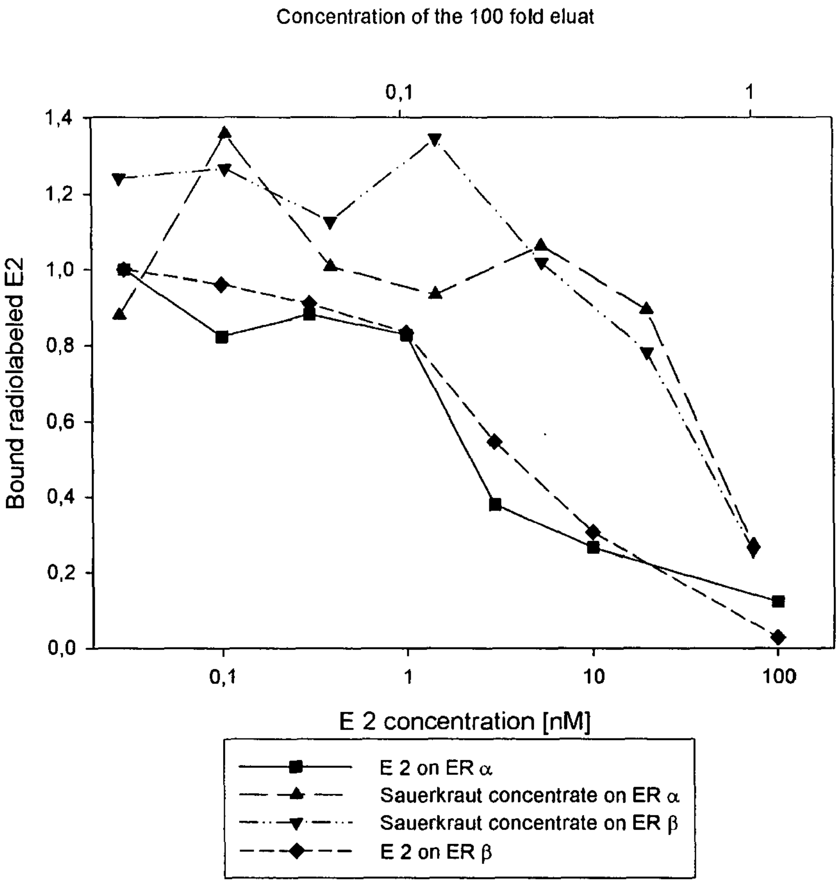
Concentration of the 100 fold eluat
0,1
1
1,4
1,2
1,0
0,8
0,6
0,4
0,2
0,0
Bound radiolabeled E2
0,1
1
10
100
E 2 concentration [nM]
E 2 on ER α
Sauerkraut concentrate on ER α
Sauerkraut concentrate on ER β
E 2 on ER β

Figure 19

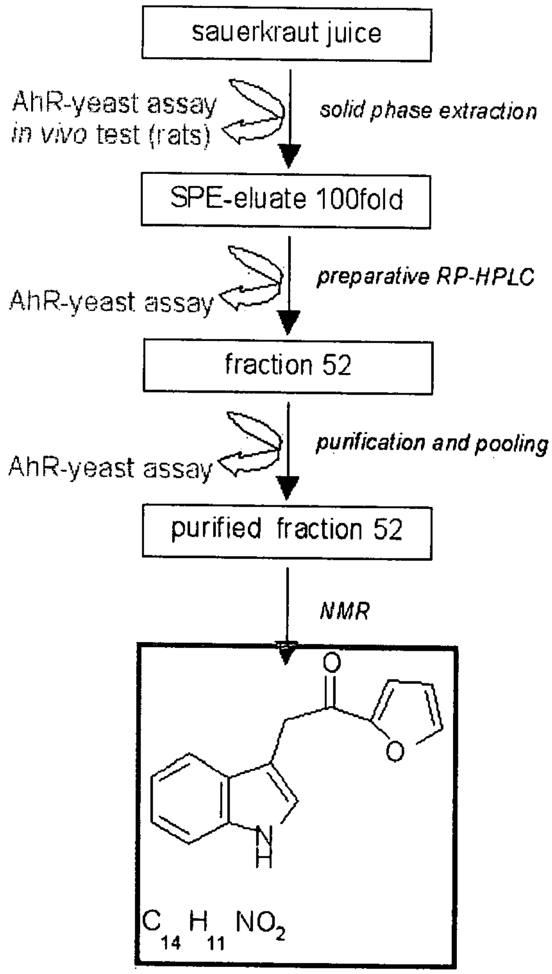
sauerkraut juice
AhR-yeast assay
*in vivo* test (rats)
*solid phase extraction*
SPE-eluate 100fold
AhR-yeast assay
*preparative RP-HPLC*
fraction 52
AhR-yeast assay
*purification and pooling*
purified fraction 52
*NMR*
$C_{14}H_{11}NO_2$

European Patent Office

## PARTIAL EUROPEAN SEARCH REPORT

which under Rule 45 of the European Patent Convention shall be considered, for the purposes of subsequent proceedings, as the European search report

Application Number
EP 06 45 0045

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | DE 35 36 342 A1 (WOLF,WALTER,DR) 8 October 1987 (1987-10-08) Claims ----- | 9-21 | INV. A61K31/404 A61K31/352 A61K31/366 A61K31/353 A61K36/31 A23L1/30 A61P15/12 A61P25/34 A61P35/00 A61P43/00 |
| X | DE 100 04 349 A1 (BUNDSCHUH, GERHARD) 2 August 2001 (2001-08-02) * the whole document * ----- | 9-21 | |
| X | DE 20 2004 013288 U1 (PHILIPP GMBH & CO KG) 18 November 2004 (2004-11-18) * paragraphs [0002], [0004], [0005], [0010], [0012], [0017] - [0020], [0026] - [0029] * Claims ----- | 9-21 | |
| X | DE 102 27 717 A1 (OKYAY, ZEKI) 19 February 2004 (2004-02-19) * the whole document * ----- | 9-21 | |
| D,X | US 2002/147155 A1 (FOSTER WARREN G ET AL) 10 October 2002 (2002-10-10) * paragraphs [0003], [0017], [0021] - [0027], [0041] - [0044] * ----- -/-- | 9-21 | TECHNICAL FIELDS SEARCHED (IPC) A61K A61P A23L |

### INCOMPLETE SEARCH

The Search Division considers that the present application, or one or more of its claims, does/do not comply with the EPC to such an extent that a meaningful search into the state of the art cannot be carried out, or can only be carried out partially, for these claims.

Claims searched completely :

Claims searched incompletely :

Claims not searched :

Reason for the limitation of the search:

see sheet C

2

| Place of search | Date of completion of the search | Examiner |
|---|---|---|
| Munich | 10 August 2006 | Hornich, E |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C07)

European Patent Office

# PARTIAL EUROPEAN SEARCH REPORT

Application Number
EP 06 45 0045

| Category | Citation of document with indication, where appropriate, of relevant passages | Relevant to claim | CLASSIFICATION OF THE APPLICATION (IPC) |
|---|---|---|---|
| X | WO 02/28832 A (THE TEXAS A & M UNIVERSITY SYSTEM) 11 April 2002 (2002-04-11)<br>* paragraphs [0007], [0008], [0017], [0023] - [0025], [0028], [0039], [0042] - [0050], [0052], [0053] *<br>* examples 7,8 *<br>Claims<br>----- | 9-21 | |
| D,X | EP 1 477 178 A (KELLY, GRAHAM EDMUND) 17 November 2004 (2004-11-17)<br>* paragraphs [0006], [0031] - [0048] *<br>Claims<br>----- | 3-6,9-21 | |
| X | BECK V ET AL: "Phytoestrogens derived from red clover: An alternative to estrogen replacement therapy?"<br>JOURNAL OF STEROID BIOCHEMISTRY AND MOLECULAR BIOLOGY, ELSEVIER SCIENCE LTD., OXFORD, GB,<br>vol. 94, no. 5, April 2005 (2005-04), pages 499-518, XP004876959<br>ISSN: 0960-0760<br>* the whole document *<br>----- | 3-6,9-21 | TECHNICAL FIELDS SEARCHED (IPC) |
| A | LYSENKOVA, L. N. ET AL: "Study of the transformations of 2-C-(indol-3-yl)methyl-.alpha.-L-xylo-hex-3-ulofuranosonic acid (the open form of ascorbigen) in an acidic medium"<br>RUSSIAN CHEMICAL BULLETIN (TRANSLATION OF IZVESTIYA AKADEMII NAUK, SERIYA KHIMICHESKAYA) , 50(7), 1309-1313 CODEN: RCBUEY; ISSN: 1066-5285, 2001, XP009070933<br>* the whole document *<br>----- | | |

2

EPO FORM 1503 03.82 (P04C10)

European Patent Office

**INCOMPLETE SEARCH SHEET C**

Application Number
EP 06 45 0045

Although claim 21 is directed to a method of treatment of the human/animal body (Article 52(4) EPC), the search has been carried out and based on the alleged effects of the compounds.

-----

**ANNEX TO THE EUROPEAN SEARCH REPORT ON EUROPEAN PATENT APPLICATION NO.** EP 06 45 0045

This annex lists the patent family members relating to the patent documents cited in the above-mentioned European search report.
The members are as contained in the European Patent Office EDP file on
The European Patent Office is in no way liable for these particulars which are merely given for the purpose of information.

10-08-2006

| Patent document cited in search report | Publication date | Patent family member(s) | Publication date |
|---|---|---|---|
| DE 3536342 A1 | 08-10-1987 | NONE | |
| DE 10004349 A1 | 02-08-2001 | NONE | |
| DE 202004013288 U1 | 18-11-2004 | NONE | |
| DE 10227717 A1 | 19-02-2004 | NONE | |
| US 2002147155 A1 | 10-10-2002 | WO 02080906 A1 | 17-10-2002 |
| WO 0228832 A | 11-04-2002 | AU 1978202 A<br>CA 2425281 A1<br>EP 1328513 A2 | 15-04-2002<br>11-04-2002<br>23-07-2003 |
| EP 1477178 A | 17-11-2004 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 20020147155 A1
- US 5948808 A
- US 20030096855 A
- EP 1418164 A1
- EP 1477178 A2
- US 20030219499 A1
- EP 1174144 A1
- WO 9943335 A


### Non-patent literature cited in the description


- **BURBACH et al.** *Proc Natl Acad Sci U S A,* 1992, vol. 89, 8185-8189
- **AMAKURA et al.** *Biol Pharm Bull,* 2003, vol. 26, 1754-1760
- **AMAKURA et al.** *Biol Pharm Bull,* 2002, vol. 25, 272-274
- **JEUKEN et al.** *J Agric Food Chem,* 2003, vol. 51, 5478-5487
- **ASHIDA et al.** *Biofactors,* 2000, vol. 12, 201-206
- **ZHANG et al.** *Environ Health Perspect,* 2003, vol. 111, 1877-1882
- **ZHOU et al.** *Mol Pharmacol,* 2003, vol. 63, 915-924
- **HAHN et al.** *Mar Biotechnol (NY,* 2001, vol. 3, 224-238
- **MIMURA et al.** *Biochim Biophys Acta,* 2003, vol. 1619, 263-268
- **PETRULIS et al.** *J Biochem Mol Toxicol,* 2000, vol. 14, 73-81
- **SWANSON et al.** *J Biol Chem,* 1995, vol. 270, 26292-26302
- **DENISON et al.** *Toxicol,* 2003, vol. 43, 309-334
- **MILLER III et al.** *Toxicology and Applied Pharmacology,* 1999, vol. 160, 297-303
- **KIPPERT et al.** *FEMS Microbiol Lett,* 1995, vol. 128, 201-206
- **ADACHI et al.** *Journal of Biological Chemistry,* 2001, vol. 276, 31475-31478
- **BECK et al.** *J Steroid Biochem Mol Biol,* 2003, vol. 84, 259-268
- **SAKAKIBARA et al.** *Biofactors,* 2004, vol. 22, 237-239
- **JUNGBAUER et al.** *Journal of Chromatography B: Analytical Technologies in the Biomedical and Life Sciences,* 2002, vol. 777, 167-178
- **KOSTELAC et al.** *J Agric Food Chem,* 2003, vol. 51, 7632-7635
- **PROMBERGER et al.** *J Agric Food Chem,* 2001, vol. 49, 633-640
- **CISKA et al.** *J Agric Food Chem,* 2004, vol. 52, 7938-7943
- **TOLONEN et al.** *J Agric Food Chem,* 2002, vol. 50, 6798-6803
- **CHEN et al.** *Carcinogenesis,* 1998, vol. 19, 1631-1639
- **KRENN et al.** *J Chromatogr B Analyt Technol Biomed Life Sci,* 2002, vol. 777, 123-128
- **PANOSSIAN et al.** *Phytochem Anal,* 2004, vol. 15, 100-108
- **FORSYTHE et al.** *Mol Cell Biol,* 1996, vol. 16, 4604-4613
- **SHAMS et al.** *Acad Sci U S A,* 2004, vol. 101, 9698-9703
- **POLLENZ et al.** *Mol Pharmacol,* 1999, vol. 56, 1127-1137
- **KOZAK et al.** *Dev Biol,* 1997, vol. 191, 297-305
- **KASSIE et al.** *Chem-Biol Interact,* 2003, vol. 142, 285-296
- **KASSIE et al.** *Carcinogenesis,* 2002, vol. 23 (7), 1155-1161
- **FREYBERGER ; AHR.** *Toxicology,* 2004, vol. 195 (2-3), 113-126
- **KLINGE et al.** *Journal of Agricultural and Food Chemistry,* 2003, vol. 51 (7), 1850
- **LYSENKOVA et al.** *Russian Chemical Bulletin, Int. Ed.,* 2001, vol. 50 (7), 1309
- **LAVRENOV et al.** *Pharmaceutical Chemistry Journal,* 2005, vol. 39 (5), 251